(19) Europäisches Patentamt
European Patent Office
Office européen des brevets

(11) EP 2 004 757 B1

(12) **FASCICULE DE BREVET EUROPEEN**

(45) Date de publication et mention de la délivrance du brevet:
**04.05.2011 Bulletin 2011/18**

(21) Numéro de dépôt: **07731815.2**

(22) Date de dépôt: **23.03.2007**

(51) Int Cl.:
***C09B 23/14*** *(2006.01)* ***C09B 49/12*** *(2006.01)*
***A61Q 5/08*** *(2006.01)* ***A61Q 5/06*** *(2006.01)*
***A61K 8/49*** *(2006.01)*

(86) Numéro de dépôt international:
**PCT/FR2007/051003**

(87) Numéro de publication internationale:
**WO 2007/110537 (04.10.2007 Gazette 2007/40)**

(54) **COMPOSITION DE TEINTURE COMPRENANT UN COLORANT FLUORESCENT THIOL/ DISULFURE A GROUPES AMINES ET A CHARGE CATIONIQUE INTERNE, PROCEDE D'ECLAIRCISSEMENT DES MATIERES KERATINIQUES A PARTIR DE CE COLORANT**

FÄRBEZUSAMMENSETZUNG MIT EINEM THIOL/DISULFID- LEUCHTFARBSTOFF MIT AMINGRUPPEN UND EINER INTERNEN KATIONISCHEN LADUNG SOWIE VERFAHREN ZUR AUFHELLUNG VON KERATINMATERIAL MIT DIESEM FARBSTOFF

DYEING COMPOSITION CONTAINING A THIOL/DISULPHIDE FLUORESCENT COLORANT COMPRISING AMINE GROUPS AND HAVING AN INTERNAL CATIONIC CHARGE, AND METHOD FOR LIGHTENING KERATIN MATERIALS USING SAID COLORANT

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **24.03.2006 FR 0651035**
**19.04.2006 US 792941 P**
**05.02.2007 FR 0753070**
**09.02.2007 US 900361 P**

(43) Date de publication de la demande:
**24.12.2008 Bulletin 2008/52**

(73) Titulaire: **L'Oréal**
**75008 Paris (FR)**

(72) Inventeurs:
- **GREAVES, Andrew**
**F-77144 Montevrain (FR)**
- **DAUBRESSE, Nicolas**
**F-78170 La Celles St Cloud (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 647 580** **EP-A1- 0 860 636**
**WO-A-03/028685** **WO-A-2005/004822**
**WO-A-2006/134043** **WO-A-2006/136617**
**WO-A2-2004/091473** **WO-A2-2005/097051**
**GB-A- 2 180 215** **US-A- 2 904 385**

- **GEOFFREY J. ASHWELL, ABDUL MOHIB AND JAMES R. MILLER: "Induced rectification from self-assembled monolayers of sterically hindered -bridged chromophores" JOURNAL OF MATERIALS CHEMISTRY, vol. 15, no. 11, 27 janvier 2005 (2005-01-27), pages 1160-1166, XP002419175**
- **GEOFFREY J. ASHWELL AND ABDUL MOHIB: "Improved Molecular Rectification from Self-Assembled Monolayers of a Sterically Hindered Dye" J. OF THE AMERICAN CHEMICAL SOCIETY, vol. 127, no. 46, 11 janvier 2005 (2005-01-11), pages 16238-16244, XP002419176**
- **GEOFFREY J. ASHWELL, WAYNE D. TYRRELL AND ANNE J. WHITTAM: "Molecular rectification: self-assembled monolayers of a donor?(-bridge)?acceptor chromophore connected via a truncated Au?S?(CH2)3 bridge" JOURNAL OF MATERIALS CHEMISTRY, vol. 13, no. 12, 3 novembre 2003 (2003-11-03), pages 2855-2857, XP002419177**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne la coloration de matières kératiniques à l'aide de colorants fluorescents thiols/disulfures comprenant un ou plusieurs groupes aminés.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triaryl méthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Par ailleurs, la coloration des fibres kératiniques à partir de colorants directs classiques ne permet pas d'éclaircir de façon notable les fibres kératiniques.

L'éclaircissement de la couleur de fibres kératiniques, plus particulièrement foncées vers des nuances plus claires, en modifiant éventuellement la nuance de celles-ci, constitue une demande importante.

Classiquement, pour obtenir une coloration plus claire on met en oeuvre un procédé de décoloration chimique. Ce procédé consiste à traiter les matières kératiniques telles que les fibres kératiniques, notamment les cheveux, par un système oxydant fort, généralement constitué par du peroxyde d'hydrogène associé ou non à des persels, le plus souvent en milieu alcalin.

Ce système de décoloration présente l'inconvénient de dégrader des matières kératiniques, en particulier les fibres kératiniques, notamment humaines telles que les cheveux et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres particulièrement dans les traitements de défrisage.

Une autre technique d'éclaircissement consiste à appliquer sur les cheveux foncés des colorants directs fluorescents. Cette technique décrite notamment dans les documents FR 2830189 et WO 2004/091473 permet de respecter la qualité de la fibre kératinique lors du traitement mais les colorants fluorescents employés ne présentent pas une résistance aux shampoings satisfaisante.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres kératiniques voir par exemple Journal of the Society of Dyers and Colourists, Guise et Stapleton, 91, 259-264 (1975); Journal of Cosmetic Chemistry, 42, 1-17 (1991); CA 2024509;

De plus il est connu de protéger la ou les fonctions thiols contenues dans une molécule à greffer aux cheveux avant de les appliquer auxdits cheveux WO 99/51194. Cependant cette demande ne mentionne pas l'utilisation de colorants fluorescents permettant de colorer ou d'éclaircir des cheveux.

D'autres colorants disulfures connus pour la coloration des fibres kératiniques sont des dérivés disulfures de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un prétraitement réducteur du cheveu. Cependant, ces colorants peuvent occasionner des virages de couleur lors de l'application.

Enfin, le document WO 2005/097051 décrit des colorants disulfures aza-imidazoliums pour la coloration directe de fibres kératiniques.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants. En particulier, un des buts de la présente invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Ce but est atteint avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent thiol, choisi parmi les colorants de formules (I) et (II) suivantes :

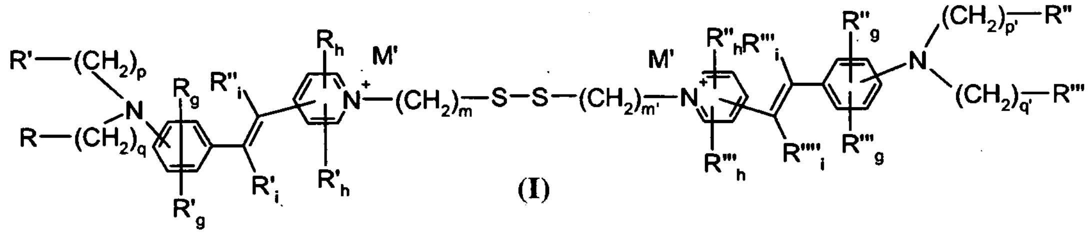

(I)

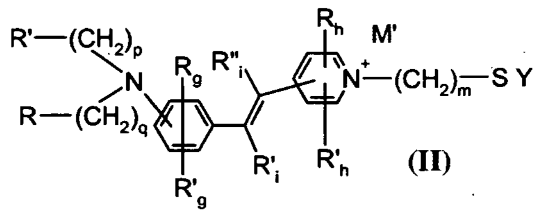

(II)

leurs sels d'acide organique ou minéral, isomères optiques, isomères géométriques, et les solvates tels que hydrates ; formules (I) et (II) dans lesquelles :

➢ R et R''', identiques ou différents, représentent un groupement hydroxy, un groupement amino ($NR_aR_b$) ou ammonium ($N^+R_aR_bR_c$), $An^-$; avec $R_a$, $R_b$ et $R_c$, identiques ou différents, représentant un atome d'hydrogène ou un groupement ($C_1$-$C_4$)alkyle ; ou alors deux groupes alkyle $R_a$ et $R_b$ du groupement amino ou ammonium forment un hétérocycle comprenant de 5 à 7 chaînons et comprenant éventuellement un autre hétéroatome identique ou différent à celui de l'atome d'azote tel que morpholinyle, pipérazinyle, pipéridinyle, pyrrolyle, morpholinium, pipérazinium, pipéridinium, pyrrolinium, et $An^-$ représentant un contre-ion anionique ;

➢ R' et R'', identiques ou différents, représentent un atome d'hydrogène ou un groupement tel que défini pour R et R''' respectivement ;

➢ $R_g$, $R'_g$, $R''_g$, $R'''_g$, $R_h$, $R'_h$, $R''_h$ et $R'''_h$, identiques ou différents, représentent un atome d'hydrogène, d'halogène, un groupement amino, (di)($C_1$-$C_4$)alkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy, ($C_1$-$C_4$)alcoxycarbonyle, ($C_1$-$C_4$)alkylcarbonylamino, acylamino, carbamoyle , ($C_1$-$C_4$)alkylsulfonylamino, un radical aminosulfonyle, ou un radical ($C_1$-$C_{16}$)alkyle éventuellement substitué par un groupement choisi parmi ($C_1$-$C_{12}$)alcoxy, hydroxy, cyano, carboxy, amino, (di)($C_1$-$C_4$) alkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ; particulièrement $R_g$, $R'_g$, $R''_g$, $R'''_g$, $R_h$, $R'_h$, $R''_h$ et $R'''_h$ représentent un atome d'hydrogène ou un groupement ($C_1$-$C_4$)alkyle;

➢ $R'_i$, $R''_i$, $R'''_i$ et $R''''_i$, identiques ou différents, représentent un atome d'hydrogène, ou un groupement ($C_1$-$C_4$) alkyle ; particulièrement $R'_i$, $R''_i$, $R'''_i$, et $R''''_i$ représente un atome d'hydrogène ;

➢ m, m', identiques ou différents, représentent un entier compris inclusivement entre 1 et 10 ; particulièrement un entier compris inclusivement entre 2 et 4 ; préférentiellement m et m' valent 2 ;

➢ p, p', q et q', identiques ou différents, représentent un entier compris inclusivement entre 1 et 6 ;

➢ M' représentant un contre-ion anionique ; et

➢ Y représente : i) un atome d'hydrogène ; ii) un métal alcalin ; iii) un métal alcalino-terreux ; iv) un groupement ammonium: $N^+R^{\alpha}R^{\beta}R^{\gamma}R^{\delta}$ ou un groupement phosphonium : $P^+R^{\alpha}R^{\beta}R^{\gamma}R^{\delta}$ avec $R^{\alpha}$, $R^{\beta}$, $R^{\gamma}$ et $R^{\delta}$, identiques ou différents, représentant un atome d'hydrogène ou un groupe ($C_1$-$C_4$)alkyle ; ou v) un groupement protecteur de fonction thiol ;

étant entendu que :

- lorsque le composé de formule **(I)** ou **(II)** contient d'autres parties cationiques, il se trouve associé à un ou plusieurs contre-ions anioniques permettant d'atteindre l'électroneutralité de la formule **(I)** ou **(II).**

Un autre objet de l'invention est une composition tinctoriale comprenant, dans un milieu cosmétique approprié au moins un colorant fluorescent de formule **(I)** ou **(II)** tels que définis précédemment, et un agent réducteur.

L'invention a aussi pour objet de nouveaux colorants fluorescents de formule (I) ou (II) tels que définis précédemment. Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Au sens de l'invention, on entend par matière kératinique foncée celle qui présente une luminescence L* chiffrée dans le système C.I.E.L. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de « hauteur de ton » avant et après application du composé de formule **(I)** ou **(II).**

La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitement capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

De préférence, la composition doit, après application sur des cheveux, par exemple châtains, amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

- les radicaux « aryle » ou « hétéroaryle » ou la partie aryle ou hétéroaryle d'un radical peuvent être substitués par au moins un substituant porté par un atome de carbone, choisi parmi :

  - un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
  - un atome d'halogène tel que chlore, fluor ou brome ;
  - un groupement hydroxy ;
  - un radical alcoxy en $C_1$-$C_2$ ;
  - un radical alkylthio en $C_1$-$C_2$;
  - un radical (poly)-hydroxyalcoxy en $C_2$-$C_4$ ;

- un radical amino ;
- une radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  i) un groupement hydroxy,
  ii) un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

- -NR-COR' dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ;
- $(R)_2N$-CO- dans lequel les radicaux R, identiques ou non, représentent un atome d'hydrogène, un radical alkyle en $C_1$-$C_4$ éventuellement porteur d'au moins un groupement hydroxy ;
- $R'SO_2$-NR- dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ;
- $(R)_2N$-$SO_2$- dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- une groupement cyano ;
- un groupement polyhalogénoalkyle comprenant de 1 à 6 atomes de carbones et de 1 à 6 atome d'halogène, identiques ou différents, le groupement polyhalogénoalkyle est par exemple le trifluorométhyle ;

- la partie cyclique ou hétérocyclique d'un radical non aromatique peut être substituée par au moins un substituant porté par un atome de carbone choisi parmi les groupements :

  - hydroxy,
  - alcoxy en $C_1$-$C_4$,
  - (poly)hydroxyalcoxy en $C_2$-$C_4$,
  - un radical alkylthio en $C_1$-$C_2$;
  - RCO-NR'- dans lequel le radical R' est un atome d'hydrogène, un radical alkyle en $C_1$-$C_4$ éventuellement porteur d'au moins un groupement hydroxy et le radical R est un radical alkyle en $C_1$-$C_2$, amino substitué par deux groupements alkyle identiques ou différents en $C_1$-$C_4$ éventuellement porteurs d'au moins un groupement hydroxy ;
  - RCO-O- dans lequel le radical R est un radical alkyle en $C_1$-$C_4$, amino substitué par un ou deux groupements alkyle identiques ou différents en $C_1$-$C_4$ éventuellement porteurs d'au moins un groupement hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
  - RO-CO- dans lequel le radical R est un radical alkyle en $C_1$-$C_4$ éventuellement porteurs d'au moins un groupement hydroxy ;

- un radical cyclique, hétérocyclique, ou une partie non aromatique d'un radical aryle ou hétéroaryle, peut également être substitué par un ou plusieurs groupements oxo ou thioxo ;
- un radical « aryle » représente un groupement mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un radical « diarylalkyle » représente un groupement comportant sur le même atome de carbone d'un groupement alkyle deux groupement aryle, identiques ou différents tel que diphénylméthyle ou 1,1-diphényléthyle ;
- un « radical hétéroaryle » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle,

benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;

- un radical « dihétéroarylalkyle » représente un groupement comportant sur le même atome de carbone d'un groupement alkyle deux groupement hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyle pouvant former avec l'atome d'azote qui les portent un hétérocyle comprenant de 5 à 7 chaînons, comprenant éventuellement un autre hétéroatome différent ou non de l'azote ; v) ou un groupement ammonium quaternaire -N R'R"R"', M pour lequel R', R", R"', identiques ou différents représentent un atome d'hydrogène, ou un groupement alkyle en $C_1$-$C_4$, ou alors $-N^+$R'R"R"' forme un hétéroaryle tel que imidazolium éventuellement substitué par un groupement $C_1$-$C_4$ alkyle, et $M^-$ représente le contre-ion de l'acide organique, minéral ou de l'halogénure correspondant,
- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$;
- un « radical alkylthio » est un radical alkyl-S- pour lequel le radical alkyle est un radical hydrocarboné, linéaire ou ramifié, en $C_1$-$C_{16}$ préférentiellement en $C_1$-$C_8$ ; lorsque le groupement alkylthio est éventuellement substitué, cela sous entend que le groupe alkyle est éventuellement substitué tel que défini précédemment ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : $ArS(O)_2OH$ tel que d'acide benzène sulfonique et d'acide toluène sulfonique ; vi) d'acide citrique ; vii) d'acide succinique ; viii) d'acide tartrique ; ix) d'acide lactique, x) d'acides alcoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3COOH$ ; xiv) d'acide triflique $CF_3SO_3H$ et xv) d'acide tétrafluoroborique $HBF_4$ ;
- un « contre-ion anionique » est un anion ou un groupement anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate ou tosylate ; v) le citrate ; vi) le succinate ; vii) le tartrate ; viii) le lactate ; ix) les alkylsulfates : Alk-O-$S(O)O^-$ tels que le méthysulfate et l'éthylsulfate ; x) les arylsulfates : Ar-O-$S(O)O^-$ tels que le benzènesulfate et le toluènesulfate ; xi) les alcoxysulfates : Alk-O-$S(O)_2O^-$ tel que le méthoxy sulfate et l'éthoxysulfate ; xii) les aryloxysulfates : Ar-O-S $(O)_2O^-$, xiii) le phosphate ; xiv) l'acétate ; xv) le triflate ; et xvi) les borates tels que le tétrafluoroborate.

Les colorants fluorescents de formule **(I)** ou **(II)** sont des composés capables d'absorber dans le rayonnement UV ou visible à une longueur d'onde $\lambda_{abs}$ comprise entre 250 et 800 nm et capables de réémettrent dans le domaine du visible à une longueur d'onde d'émission $\lambda_{ém}$ comprise entre 400 et 800 nm.

De préférence les composés fluorescents thiols de l'invention sont des colorants capables d'absorber dans le visible $\lambda_{abs}$ comprise entre 400 et 800 nm et de réémettre dans le visible $\lambda_{ém}$ comprise entre 400 et 800 nm. Plus préférentiellement les colorants fluorescents de formule **(I)** ou **(II)** sont des colorants capables d'absorber à une $\lambda_{abs}$ comprise entre 420 nm et 550 nm et de réémettre dans le visible à une $\lambda_{ém}$ comprise entre 470 et 600 nm.

Les composés fluorescents de formule (II) de l'invention contiennent une fonction SY qui peut se trouver sous la forme covalente -S-Y ou ionique $-S^- Y^+$ selon la nature de Y et du pH du milieu.

Un mode particulier concerne les colorants fluorescents thiols à groupe de formule (II) à fonction SY où Y représente un atome d'hydrogène, ou un métal alcalin. Avantageusement Y représente un atome d'hydrogène.

Conformément à un autre mode de réalisation particulier de l'invention, dans la formule **(II)** précitée, Y est un groupement protecteur connu par l'homme du métier comme par exemple ceux décrits dans les ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Particulièrement lorsque Y représente un groupement protecteur de la fonction thiol, Y est choisi parmi les radicaux suivants :

- ■ $(C_1-C_4)$alkylcarbonyle;
- ■ $(C_1-C_4)$alkylthiocarbonyle ;
- ■ $(C_1-C_4)$a)coxycarbonyle ;
- ■ $(C_1-C_4)$alcoxythiocarbonyle ;
- ■ $(C_1-C_4)$alkylthio-thiocarbonyle;
- ■ (di) $(C_1-C_4)$ (alkyl)aminocarbonyle ;
- ■ (di) $(C_1-C_4)$ (alkyl)aminothiocarbonyle;
- ■ arylcarbonyle comme phénylcarbonyle ;
- ■ aryloxycarbonyle ;
- ■ aryl$(C_1-C_4)$alkcoxycarbonyle;
- ■ (di) $(C_1-C_4)$ (alkyl)aminocarbonyle comme diméthylaminocarbonyle ;
- ■ $(C_1-C_4)$(alkyl)arylaminocarbonyle;
- ■ carboxy ;
- ■ $SO_3^-$, $M^+$ avec $M^+$ représentant un métal alcalin tel que le sodium ou le potassium, ou alors M' de la formule (II) et $M^+$ sont absents ;
- ■ aryle éventuellement substitué tel que le phényle, dibenzosubéryle, ou 1,3,5-cycloheptatriényle,
- ■ hétéroaryle éventuellement substitué ; dont notamment l'hétéroaryle cationiques ou non, comprenant de 1 à 4 hétéroatomes suivants :

  i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;

  ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupements tels que $(C_1-C_4)$alkyle comme méthyle, ou polyhalogéno$(C_1-C_4)$alkyle comme trifluorométhyle ;

  iii) ou tricyclique ABC suivant :

  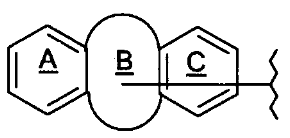

  dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

- ■ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme $(C_1-C_4)$ alkyle, oxo ou thioxo ; ou l'hétérocycle

représente le groupement suivant :

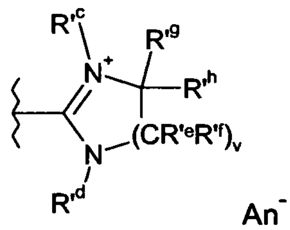

dans lequel R'$^c$, R'$^d$, R'$^e$, R'$^f$, R'$^g$ et R'$^h$, identiques ou différents représentent un atome d'hydrogène ou un groupement ($C_1$-$C_4$) alkyle, ou alors deux groupement R'$^g$ avec R'$^h$, et/ou R'$^e$ avec R'$^f$ forment un groupement oxo ou thioxo, ou alors R'$^g$ avec R'$^e$ forment ensemble un cycloalkyle ; et v représente un entier compris inclusivement entre 1 et 3 ; préférentiellement R'$^c$ à R'$^h$ représentent un atome d'hydrogène ; et $An^-$ représente un contre-ion ;

■ isothiouronium -C(NR'$^c$R'$^d$)=$N^+$R'$^e$R'$^f$; $An^-$ avec R'$^c$, R'$^d$, R'$^e$ et R'$^f$, identiques ou différents représentent un atome d'hydrogène ou un groupement ($C_1$-$C_4$)alkyle ; préférentiellement R'$^c$ à R'$^f$ représentent un atome d'hydrogène ; et $An^-$ représente un contre-ion ;

■ isothiourée -C(NR'$^c$R'$^d$)=NR'$^e$; avec R'$^c$, R'$^d$, et R'$^e$ tels que définis précédemment ;

■ (di)aryl($C_1$-$C_4$)alkyle éventuellement substitué tel que le 9-anthracénylméthyle, phénylméthyle ou diphénylméthyle éventuellement substitué par un plusieurs groupements notamment choisis parmi ($C_1$-$C_4$) alkyle, ($C_1$-$C_4$) alcoxy comme le méthoxy, hydroxy, ($C_1$-$C_4$)alkylcarbonyle, (di) ($C_1$-$C_4$) (alkyl)amino comme le diméthylamino ;

■ (di)hétéroaryl($C_1$-$C_4$)akyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupement tel que alkyle particulièrement méthyle, avantageusement le (di)hétéroaryl($C_1$-$C_4$)akyle est (di)hétéroarylméthyle ou (di) hétéroaryléthyle ;

■ $CR^1R^2R^3$ avec $R^1$, $R^2$ et $R^3$ identiques ou différents, représentant un atome d'halogène ou un groupe choisi parmi :

- ($C_1$-$C_4$) alkyle ;
- ($C_1$-$C_4$) alcoxy ;
- aryle éventuellement substitué tel que phényle éventuellement substitué par un ou plusieurs groupements comme ($C_1$-$C_4$)alkyle, ($C_1$-$C_4$)alcoxy, hydroxy ;
- hétéroaryle éventuellement substitué tel que thiophényle, furanyle, pyrrolyle, pyranyle, pyridyle, éventuellement substitué par un groupement ($C_1$-$C_4$)alkyle ;
- P($Z^1$)R'$^1$R'$^2$R'$^3$ avec R'$^1$, et R'$^2$ identiques ou différents représentent un groupement hydroxy, ($C_1$-$C_4$)alcoxy ou alkyle, R'$^3$ représente un groupement hydroxy ou ($C_1$-$C_4$)alcoxy, et $Z^1$ représente un atome d'oxygène ou de soufre ;

■ cyclique stériquement encombré tel que le groupe adamantyle ; et

■ alcoxy($C_1$-$C_4$)alkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule (II) comportent un groupement Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, , isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupements tels que alkyle comme méthyle, ou polyhalogéno($C_1$-$C_4$)alkyle comme trifluorométhyle ; iii) ou hétérocyclique suivant :

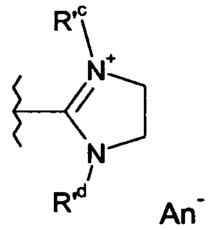

dans lequel R'c et R'd, identiques ou différents, représentent un atome d'hydrogène ou un groupement ($C_1$-$C_4$)alkyle ; préférentiellement R'c à R'd représentent un groupement ($C_1$-$C_4$)alkyle tel que méthyle ; et $An^-$ représente un contre-ion.

Particulièrement Y représente un groupement choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupements étant éventuellement substitués par un ou plusieurs groupes ($C_1$-$C_4$) alkyle notamment méthyle.

En particulier Y représente un métal alcalin ou un groupement protecteur tel que:

- ($C_1$-$C_4$)alkylcarbonyle comme méthylcarbonyle ou éthylcarbonyle ;
- arylcarbonyle comme phénylcarbonyle ;
- ($C_1$-$C_4$)alcoxycarbonyle;
- aryloxycarbonyle ;
- aryl($C_1$-$C_4$)alcoxycarbonyle;
- (di) ($C_1$-$C_4$) (alkyl)aminocarbonyle comme diméthylaminocarbonyle;
- ($C_1$-$C_4$)(alkyl)arylaminocarbonyle ;
- aryle éventuellement substitué tel que le phényle ;
- hétéroaryle monocyclique à 5 ou 6 chaînons tels que imidazolyle ou pyridyle ;
- hétéroaryle monocyclique cationique à 5, 6 chaînons tels que pyrylium, pyridinium, pyrimidinium, pyrazinium, pyridazinium, triazinium, imidazolium ; ces groupements étant éventuellement substitué par un ou plusieurs, identiques ou différents, groupes ($C_1$-$C_4$)alkyle tel que méthyle;
- hétéroaryle bicyclique cationique à 8 à 11 chaînons tels que benzoimidazolium, ou le benzoxazolium ; ces groupements étant éventuellement substitué par un ou plusieurs, identiques ou différents, groupes ($C_1$-$C_4$)alkyle tel que méthyle ;
- hétérocycle cationique de formule suivante :

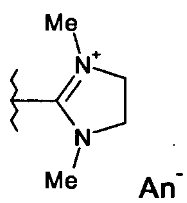

- isothiouronium $-C(NH_2)=N^+H_2$; $An^-$,
- isothiourée $-C(NH_2)=NH$ ; ou
- $SO_3^-$, $M^+$ avec $M^+$ avec $M^+$ représentant un métal alcalin tel que le sodium ou le potassium, ou alors M' de la formule (II) et $M^+$ sont absents ;.

Selon un mode particulier de l'invention, les colorants de l'invention appartiennent à la formule **(Ia)** ou **(IIa)** qui possèdent un groupement éthylène reliant la partie pyridinium au phényle en position ortho ou para du pyridinium soit en 2-4', 4-2', 4-4' :

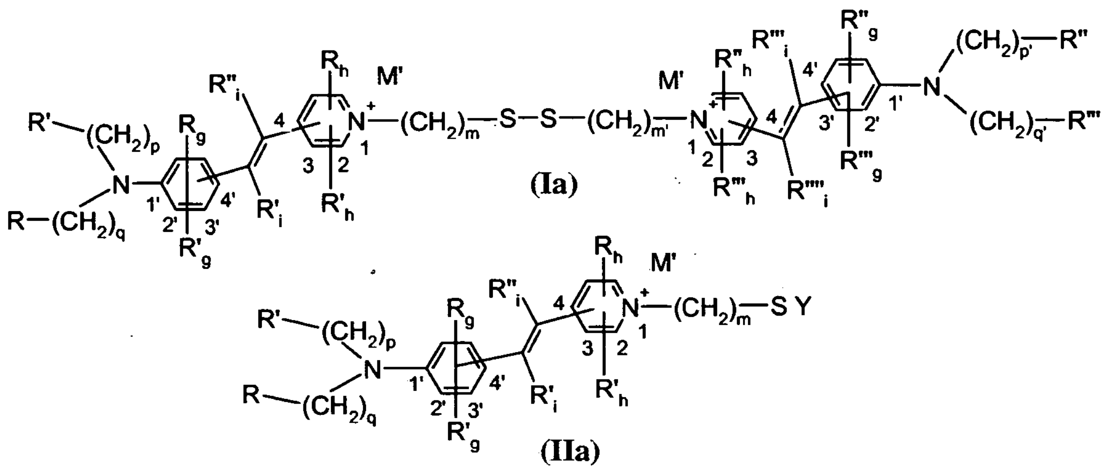

(Ia)

(IIa)

avec R, R', R'', R''', $R_g$, $R'_g$, $R''_g$, $R'''_g$, $R_h$, $R'_h$, $R''_h$, $R'''_h$, $R_i$, $R''_i$, $R'''_i$, $R''''_i$, m, m', p, p', q, q', Y et M' sont tels que définis précédemment. Particulièrement $R_h$, $R''_h$ se trouvent en ortho du groupement pyridinium et $R'_h$, $R''_h$ représentent un atome d'hydrogène. Un autre aspect de l'invention concerne les colorants de formule **(Ia)** ou **(IIa)** possédant des groupements $R_g$, $R''_g$ en position 3' et $R'_g$/$R''_g$ qui représentent un atome d'hydrogène.

Avantageusement les colorant de formule **(Ia)** et **(IIa)** possèdent leur groupement amino : $R'(CH_2)_p$-N-$(CH_2)_q$-R et/ou $R''(CH_2)_p$-N-$(CH_2)_{q'}$-R''' en position 1'.

A titre d'exemple de colorants fluorescents thiols, on peut citer notamment les composés suivants :

| |
|---|
| 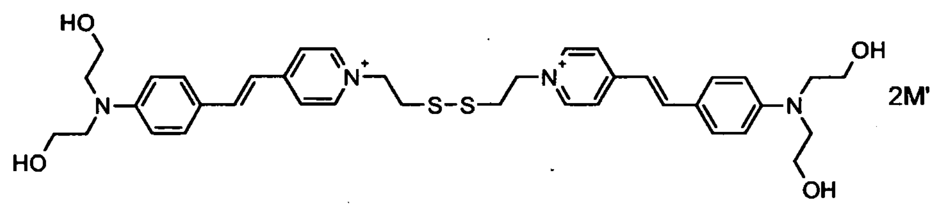 |
| 1 |
| 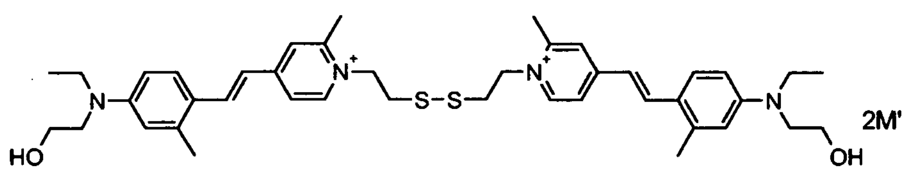 |
| 2 |
| 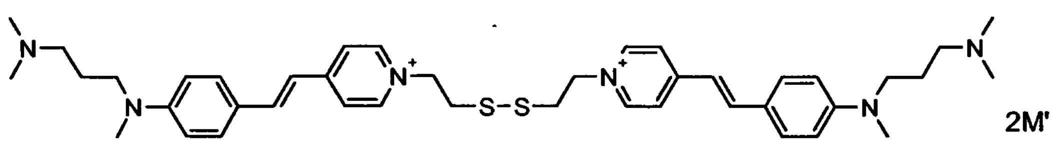 |
| 3 |

(suite)

| | |
|---|---|
| 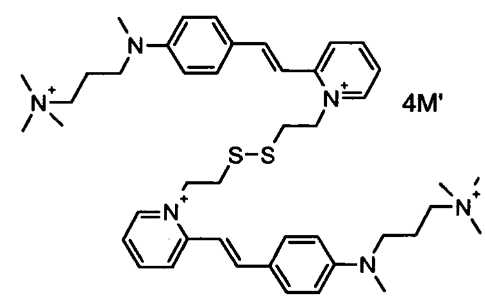 | 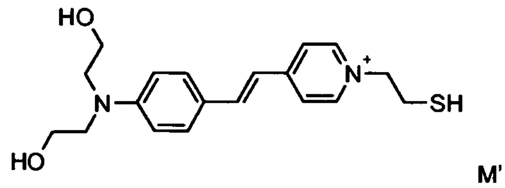 |
| 4 | 5 |
| 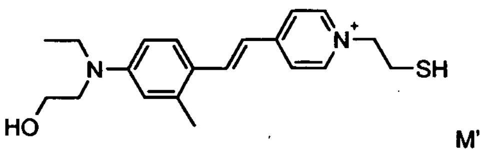 | 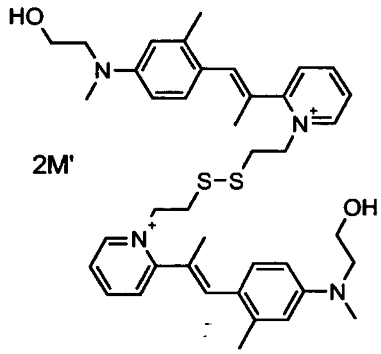 |
| 6 | 7 |
| 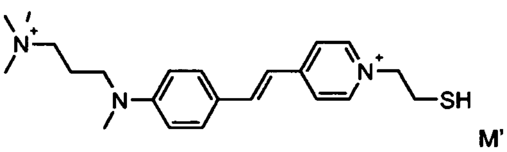 | 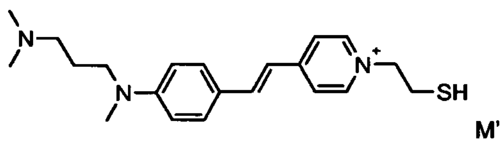 |
| 8 | 9 |
| 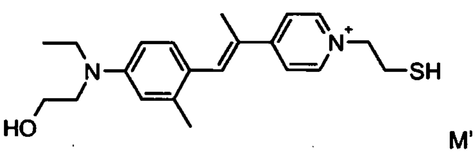 | 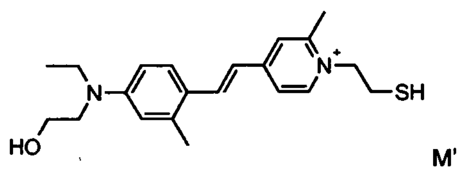 |
| 10 | 11 |
| 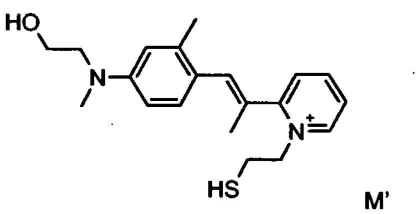 | 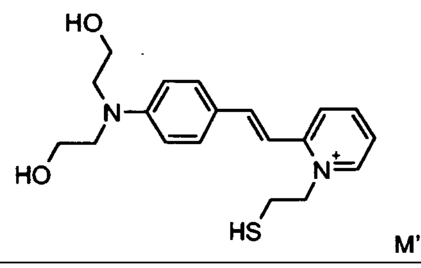 |
| 12 | 13 |

(suite)

| | |
|---|---|
| 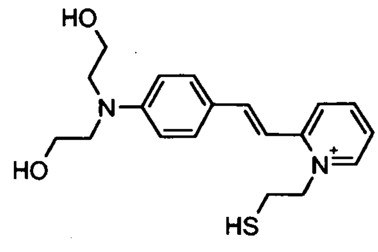 | 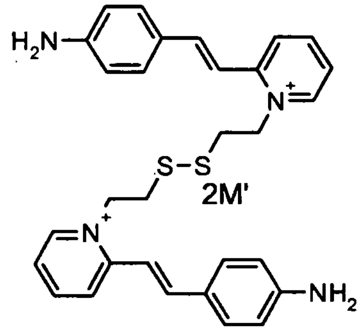 |
| **14** | **15** (n'est pas couvert par les revandications) |
| 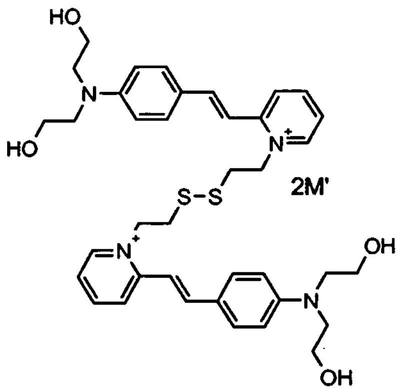 | 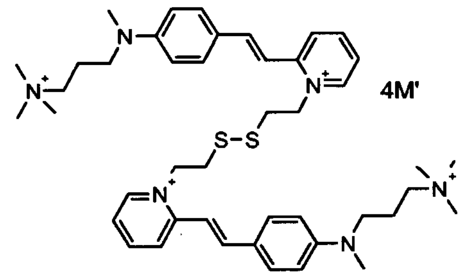 |
| **16** | **17** |
| 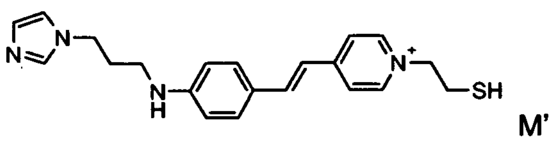 | 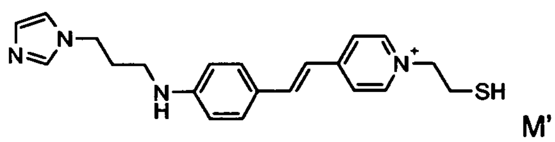 |
| **18** | **19** |
| 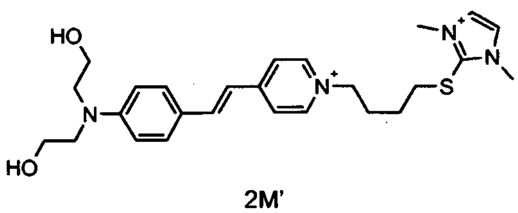 | 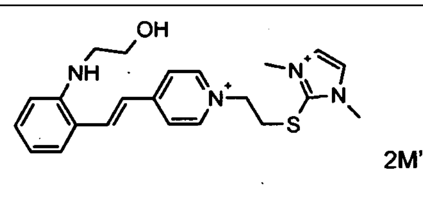 |
| **20** | **21** |
| 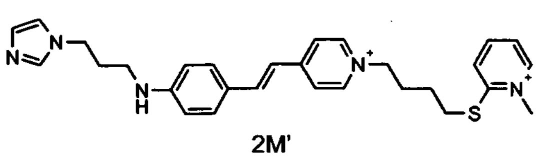 | 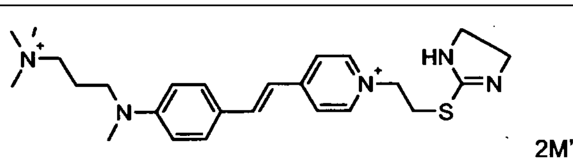 |
| **22** | **23** |

(suite)

| | |
|---|---|
| 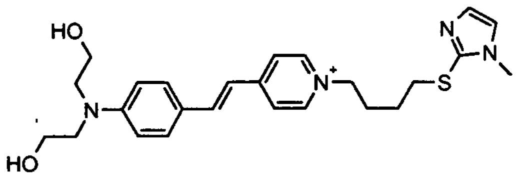 M' | 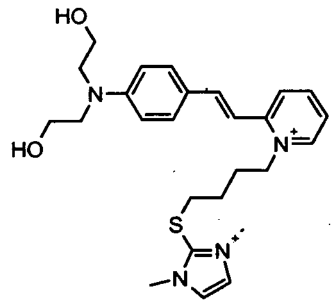 2M' |
| 24 | 25 |
| 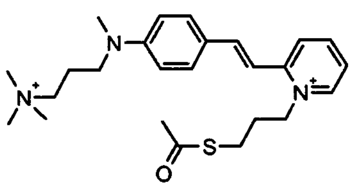 2M' | 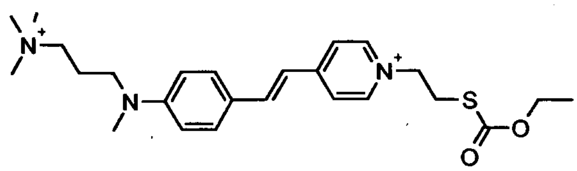 |
| 26 | 27 |
| 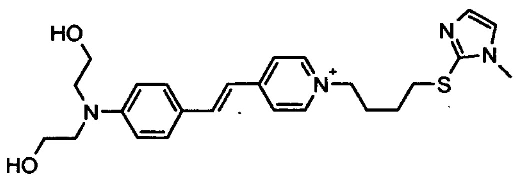 M' | 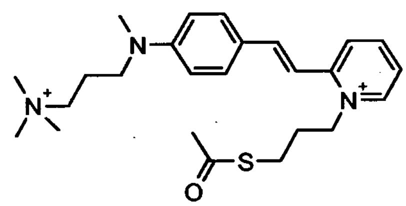 2M' |
| 28 | 29 |
| 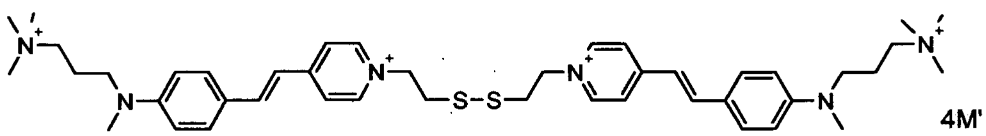 4M' | |
| 30 | |
| 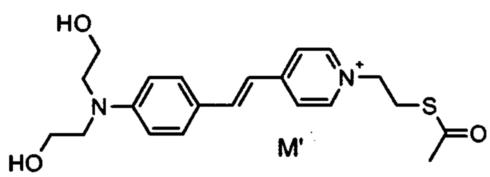 M' | |
| 31 | |
| 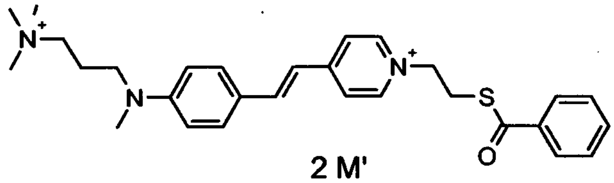 2 M' | |
| 32 | |

avec M' représentant un contre-ion anionique.

Les colorants thiols protégés de formule **(II')** peuvent être synthétisés en deux étapes. La première étape

consistant à préparer le colorant thiol non protégé **(II-H)** selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule **(II').** A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217; « Protecting Groups », P. Kocienski, Thieme, 3ème ed., 2005, chap. 5.

Nous pouvons illustrer cette méthode par la méthode consistant i) à générer des colorants fluorescents thiols de formule **(II-H)** par réduction d'un colorant fluorescent à deux chromophores, portant une fonction disulfure -S-S- (I') et ii) à protéger selon les méthodes classiques ladite fonction thiol de **(II-H)** avec le réactif **7** Y'R pour accéder au colorants fluorescents thiols protégés de formule **(II').** Le composé thiol **6** peut également être métallé avec un métal alcalin ou alcalino terreux Met* pour conduire au colorant fluorescent thiolate de formule (II").

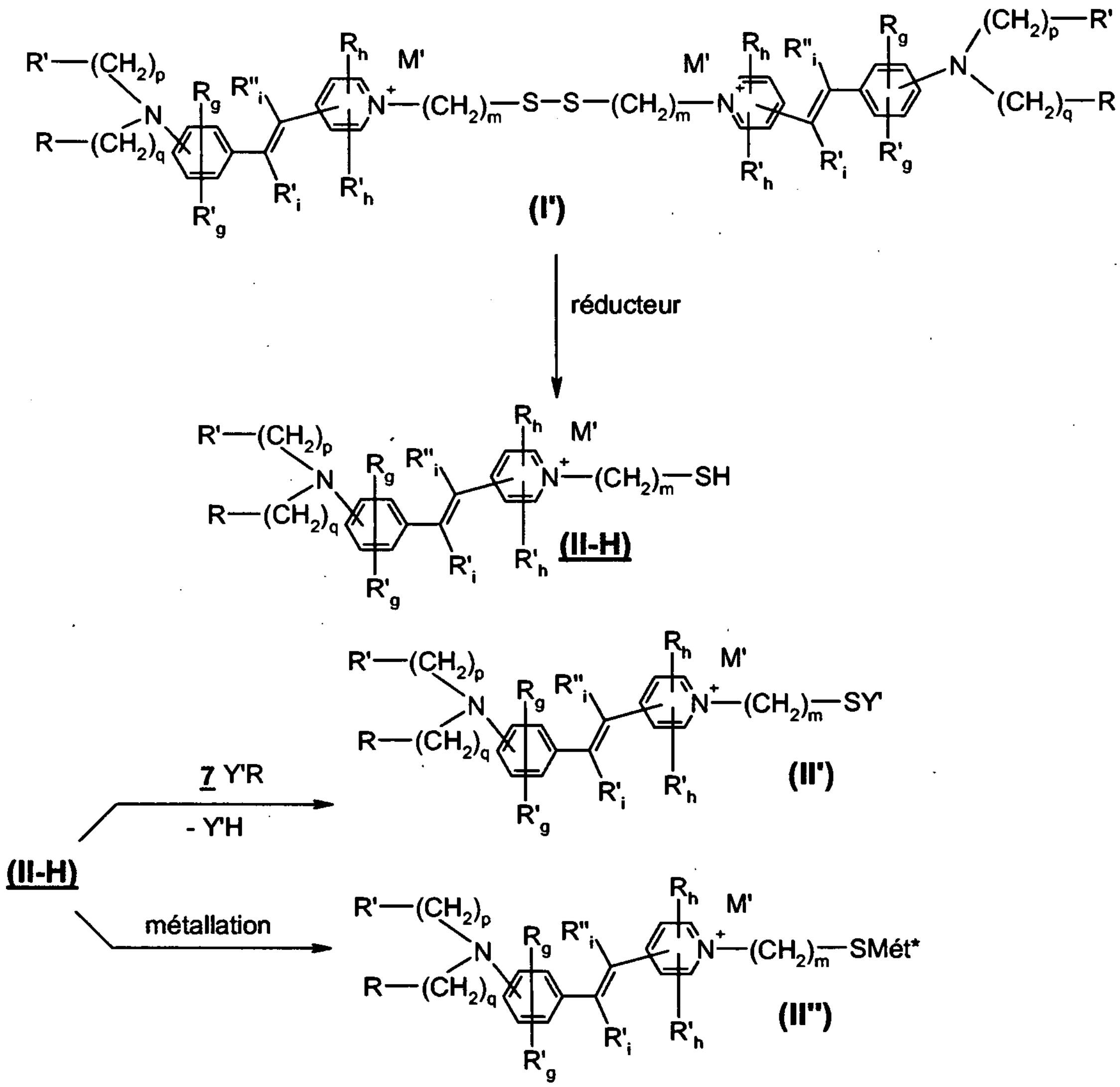


(I')

(II-H)

(II')

(II-H)

(II")

a

avec Y' représentant un groupement protecteur de fonction thiol ; Met* représentant un métal alcalin ou un métal alcalino térreux, particulièrement le sodium ou le potassium, étant entendu que lorsque le métal est un métal alkalino-terreux 2 chromophores à fonction thiolate-S- peuvent être associés à 1 Métal$^{2+}$;

et avec R, R', $R_g$, $R'_g$, $R_h$, $R'_h$, $R'_i$, $R''_i$, m, p, q, et M' sont tels que définis précédemment ; Y' représente un groupement protecteur de fonction thiol ; et R de Y'R représente un groupe partant nucléofuge, comme par exemple mésylate, tosylate, triflate ou halogénure.

On pourra également utiliser un réactif thiol Y'-SH comprenant un groupement Y' tel que défini précédemment dont la fonction nucléophile SH peut réagir sur l'atome de carbone en alpha de l'atome d'halogène porté par le chromophore fluorescent **(a')**
pour conduire au colorant fluorescent thiol protégé de formule (II'):

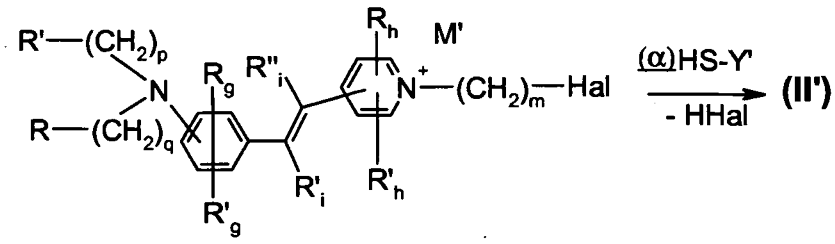


avec R, R', $R_g$, $R'_g$, $R_h$, $R'_h$, $R'_i$, $R''_i$, m, p, q, et M', (II') sont tels que définis précédemment, et Hal représentant un atome d'halogène nucléofuge tel que le brome, l'iode ou le chlore.

Plus particulièrement on pourra substituer un groupement partant nucléofuge par un groupement thiourée (S=C(NRR)NRR) pour générer les isothiouroniums. Par exemple si le groupement thiourée est une thioimidazolinium (β), le schéma réactionnel est le suivant :

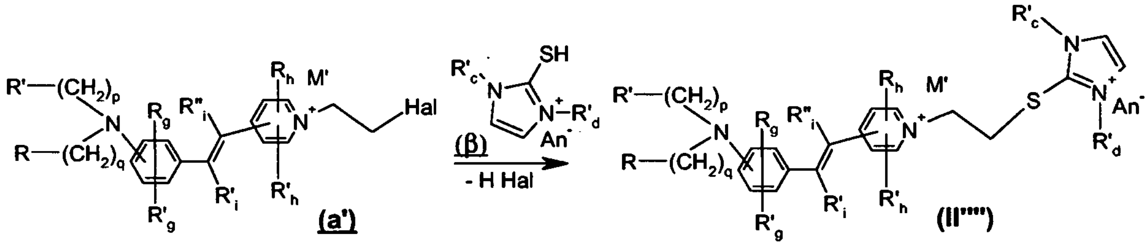


avec R, R', $R_g$, $R'_g$, $R_h$, $R'_h$, $R'_i$, $R''_i$, m, p, q, et M' sont tels que définis précédemment. Une variante est d'utiliser à la place de l'halogénure comprenant le chromophore fluorescent **(a')** un chromophore comprenant un autre type de nucléofuge tel que le tosylate, mésylate.

A titre d'exemple un composé contenant un groupement thiol protégé, contient un groupement partant nucléofuge R, comme par exemple mésylate, tosylate, ou triflate qui peut subir l'attaque nucléophile de l'amine portée par le chromophore fluorescent styrylique :

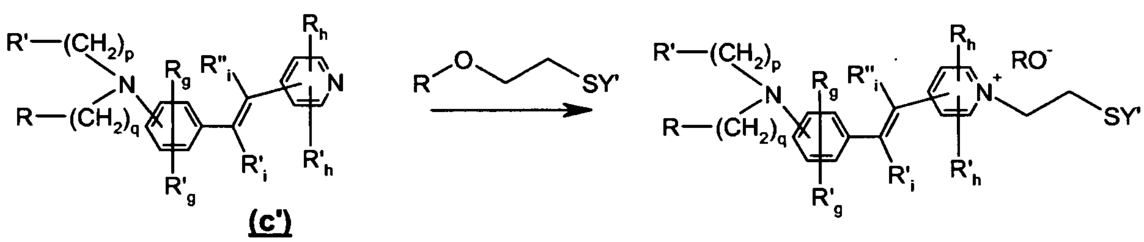


Une autre alternative provient de l'utilisation d'halogénures comme groupement partant nucléofuge sur un composé thiol pouvant être substitué par une fonction amine primaire par exemple portée par un chromophore fluorescent styrylique :

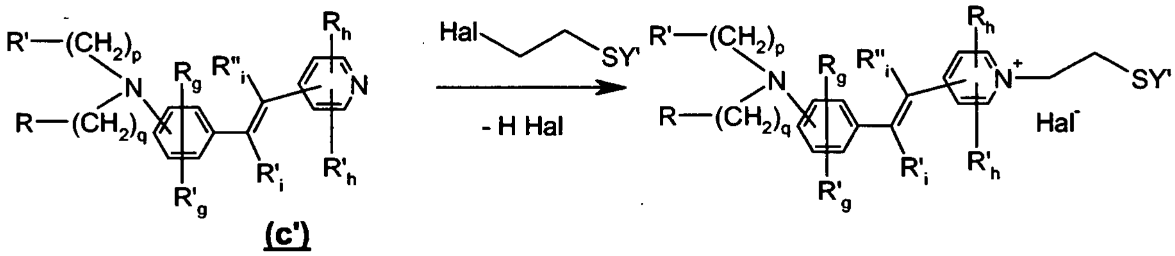


**(c')**

Conformément à une autre possibilité, les colorants fluorescents thiols de formule (II) selon l'invention peuvent être obtenus par réaction d'un composé comprenant un groupement thiol Y tel que défini précédemment et un groupement électrophile **(f)** avec un composé pyridinium comprenant un groupement nucléophile. A titre d'exemple, on pourra condenser un aldéhyde, cétone ou un thioaldéhyde, thiocétone lorsque G' représente un atome d'oxygène ou un soufre avec un « méthylène activé » tel que l'alkylpyridinium **(e)** pour générer un liaison éthylène >C=C<. Cette réaction est communément appelée condensation de « Knoevenagel ». Par « méthylènes activés » on sous entend ceux qui comporte préférentiellement en position 2 ou 4 du groupement pyridinium un groupement méthylène $R_i$-$CH_2$-:

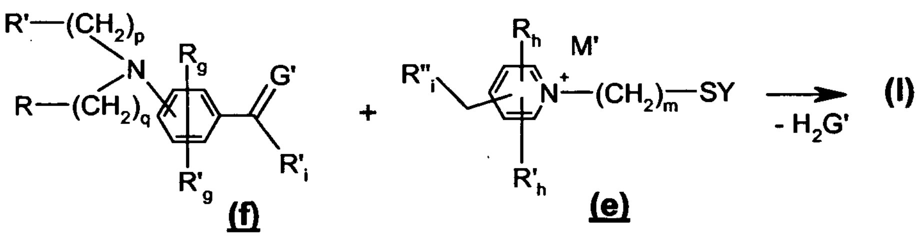


**(f)** **(e)**

avec R, R', $R_g$, $R'_g$, $R_h$, $R'_h$, $R'_i$, $R''_i$, m, p, q, Y et M' tels que définis précédemment et G' représentant un atome d'oxygène ou de soufre.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par les méthodes classiques connues par l'homme du métier. A titre d'exemple il est possible de synthétiser le colorant fluorescent thiol symétrique **(I')** à partir de 2 équivalents de dérivé pyridinique **1** et un équivalent de réactif disulfure comprenant deux groupes partants Gp, pour conduire au sel de dipyridinium disulfure **3** qui peut se condenser à son tour avec deux équivalents de composé aryle à groupement aldéhyde/thioaldéhyde **4** pour conduire à **(I')**.

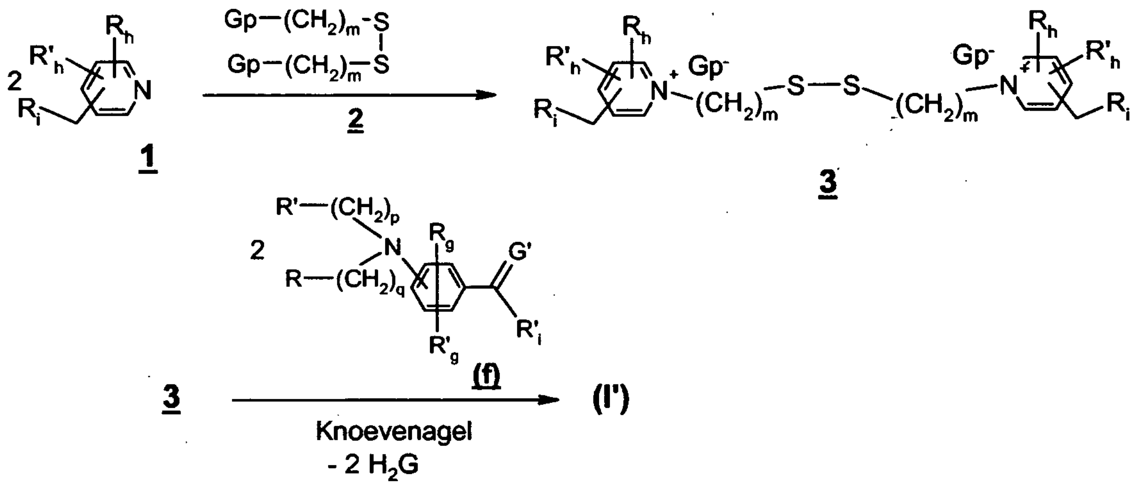


avec Gp représentant un groupe partant nucléofuge, comme par exemple mésylate, tosylate, triflate ou halogénure. Les contre-ions $Gp^-$ des composés **(I')**, ci-dessus peuvent être remplacés par des contre-ions M' d'autres natures à partir de méthodes connues par l'homme du métier notamment par résine échangeuse d'ion.

Les colorants disulfures disymétriques de formule **(I)** peuvent être synthétisés en une étape en faisant réagir un colorant fluorescent thiol non protégé avec un colorant fluorescent thiol protégé par Y' pour former le colorant disulfure de formule **(I).**

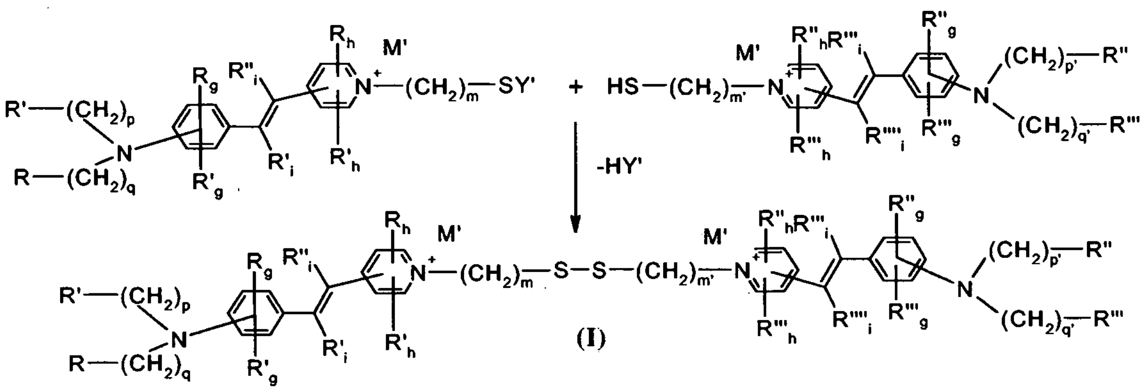


avec R, R', R", R"', $R_g$, $R'_g$, $R''_g$, $R'''_g$, $R_h$, $R'_h$, $R''_h$, $R'''_h$, $R_i$, $R'_i$, $R''_i$, $R'''_i$, m, m', p, p', q, q' et M' sont tels que définis précédemment ; Y' représente un groupement protecteur de fonction thiol.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupements protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

La composition de l'invention contient au moins un colorant fluorescent de formule **(I)** ou **(II).** Outre la présence d'au moins un colorant fluorescent de formule **(I)** ou **(II),** la composition de l'invention peut également contenir un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et

leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorant fluorescent de formule (I) ou (II) comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

Selon une variante, l'invention contient un agent réducteur capable de réduire les liaisons disulfures de la kératine et/ou du colorant de formule **(I).** Cet agent réducteur est tel que défini précédemment.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5

et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (y) suivante :

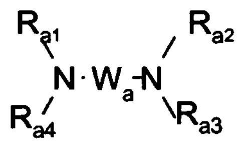 (γ)

dans laquelle $W_a$ est un reste propylène éventuellement substitué par un groupement hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Selon un mode de réalisation particulier dans le procédé de l'invention un agent réducteur peut être appliqué en pré-traitement avant l'application de la composition contenant au moins un colorant fluorescent de formule **(I)** ou **(II).**

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 0,1 seconde à 30 minutes, de préférence de 1 minute à 15 minutes avec un agent réducteur tel que cité précédemment.

Selon un autre procédé, la composition comprenant au moins un colorant fluorescent de formule **(I)** ou **(II)** contient également au moins un agent réducteur tel que défini précédemment. Cette composition est alors appliquée aux cheveux.

Lorsque le colorant fluorescent thiol de formule **(II)** comprend un groupement Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupement protecteur en ajustant le pH comme suit :

| Y : groupe protecteur | déprotection |
|---|---|
| | |
| alkylcarbonyle, | pH>9 |
| arylcarbonyle, | pH>9 |
| alcoxycarbonyle, | pH>9 |
| aryloxycarbonyle, | pH>9 |
| arylalcoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cour d'une étape de prétraitement des cheveux comme par exemple, le prétraitement réducteur des cheveux.

Selon une variante, l'agent réducteur est ajouté à la composition tinctoriale contenant au moins un colorant fluorescent de formule **(I)** ou **(II)** au moment de l'emploi.

Selon un autre procédé, la composition comprenant au moins un colorant fluorescent de formule **(I)** ou **(II)** contient également au moins un agent réducteur tel que défini précédemment. Cette composition est alors appliquée aux cheveux.

Selon une autre variante, l'agent réducteur est appliqué en post-traitement, après l'application de la composition contenant au moins un colorant fluorescent de formule **(I)** ou **(II).** La durée du post traitement avec l'agent réducteur peut être courte, par exemple de 0,1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule **(I)** ou **(II)** peut être appliqué directement aux cheveux sans réducteurs, exempt de pré ou post-traitement réducteurs.

Un traitement avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule **(1)** ou **(II).**

L'application de la composition tinctoriale selon l'invention est généralement effectuée à température ambiante. Elle peut cependant être réalisée à des températures variant de 20 à 180°C.

L'invention a aussi pour objet un dispositif à plusieurs compartiments ou "kit" de teinture dans lequel un premier compartiment renferme une composition tinctoriale comprenant au moins un colorant fluorescent de formule **(I)** ou **(II)** et un deuxième compartiment renferme un agent réducteur capable de réduire les fonctions disulfures des matières kératiniques.

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule **(I)** ou **(II) ;** un deuxième compartiment contient un agent réducteur capable de réduire la liaison disulfure des matières kératiniques ; un troisième compartiment contient un agent oxydant.

Alternativement, le dispositif de teinture contient un premier compartiment renfermant une composition tinctoriale qui comprend au moins un colorant fluorescent thiol protégé de formule **(II)** et un deuxième compartiment renfermant un agent capable de déprotéger le thiol protégé pour libérer le thiol.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents thiols des exemples ci-après ont été entièrement caractérisés par les méthodes spectroscopiques et spectrométriques classiques.

## EXEMPLES

## EXEMPLES DE SYNTHESE

### Exemple 1 : Synthèse du diméthane sulfonate de 1,1'-(disulfanedlyldiéthane-2,1-diyl)bis[4-((E)-2-(4-[bis(2-hydroxyéthyl)amino]phényl}vinyl)pyridinium] [1]

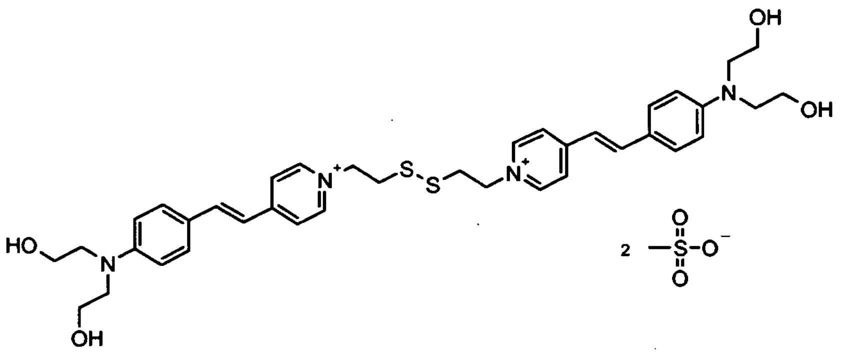

[1]

**Schéma de synthèse**

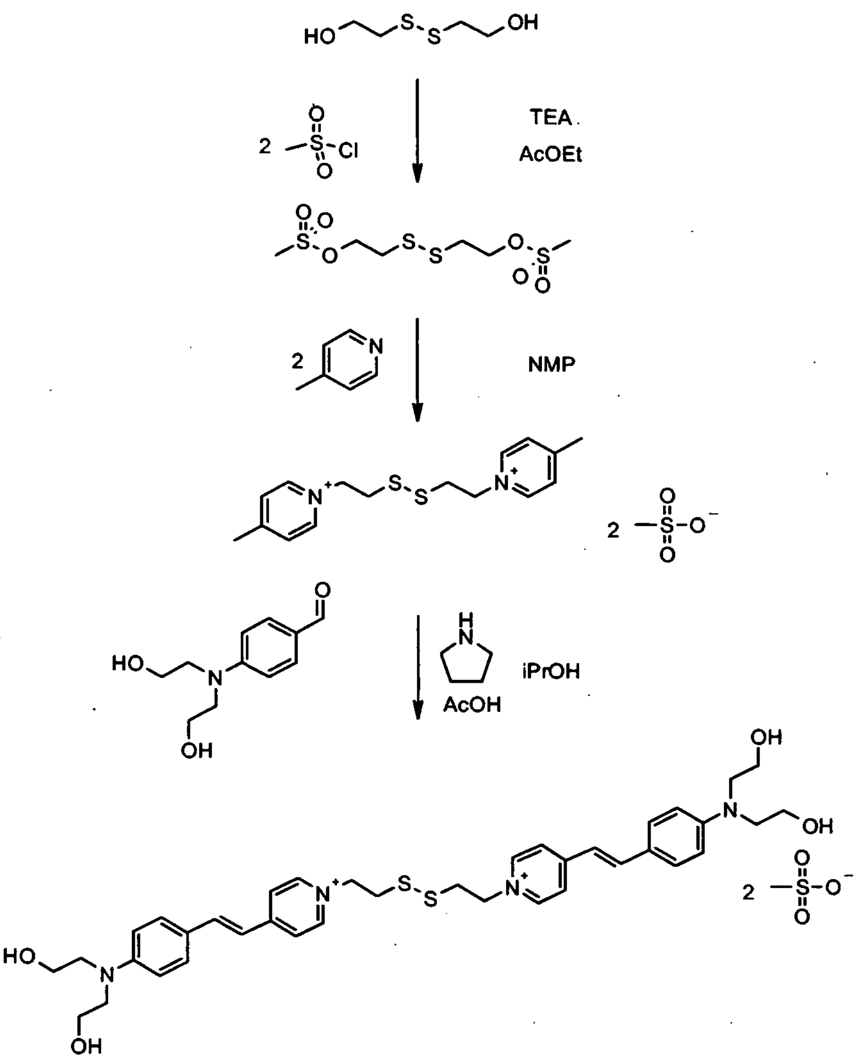


[1]

**Mode opératoire**

**Etape 1 : Synthèse du disulfanediyldiéthane-2,1-diyl diméthanesulfonate**

10 g de 2,2'-dithiodiéthanol et 14,44 g de triéthylamine (TEA) sont dilués dans 100 mL d'AcOEt. A 0 °C, 16.35 g de chlorure de méthanesulfonyle dilués dans 35 mL d'AcOEt sont ajoutés goutte à goutte au milieu réactionnel sous agitation rapide. 7,22 g de TEA sont introduits, l'agitation est poursuivie à température ambiante pendant 4 h 30. 8,2 g de chlorure de méthanesulfonyle sont ajoutés goutte à goutte à 15 °C, puis l'agitation est maintenue à température ambiante pendant 17 h. Le précipité est filtré et lavé par 3 fois 50 mL d'AcOEt. Les phases organiques sont extraites par 100 mL d'eau glacée ; 100 mL d'eau, par 3 fois 50 mL d'une solution saturée de $NaHCO_3$, par 2 fois 20 mL de NaCl saturé, puis sont séchées sur $Na_2SO_4$ anhydre. L'AcOEt est évaporé, et 17,49 g d'huile translucide jaune pâle sont recueillis. Les analyses indiquent que le produit est conforme.

**Etape 2 : Synthèse du diméthanesulfonate de 1,1'-(disulfanediyldiéthane-2,1-diyl)bis(4-méthylpyridinium)**

3,51 g de 4 picoline et 5 g de disulfanediyldiéthane-2,1-diyl diméthanesulfonate sont dilués dans 5 mL de N-méthylpyrrolidinone (NMP) puis chauffés à 80 °C sous agitation pendant 2 h. L'agitation est maintenue à température ambiante pendant 17 h. Le milieu réactionnel est complété par 50 mL d'AcOEt, puis filtré, lavé par 3 fois 100 mL d'AcOEt, et séché sous vide en présence de $P_2O_5$. 7,29 g de poudre marron sont recueillis. Les analyses indiquent que le produit est conforme et pur. RMN $^1H$ (400 MHz, DMSO-$d_6$) 2.31 (s, 6 H), 2.62 (s, 6 H), 3.40 (t, 4 H), 4.83 (t, 4 H), 8.01 (d, 4 H), 8.91 (d, 4 H).

**Etape 3 : Synthèse du diméthane sulfonate de 1,1'-(disulfanediyldiéthane-2,1-diyl)bis[4-((E)-2-{4-[bis(2-hydroxyéthyl)amino]phényl}vinyl)pyridinium] [1]**

4,32 g de 4-[bis-(2-hydroxy-éthyl)-amino]-benzaldéhyde, 10 mL d'iPrOH, et 1,69 mL de pyrrolidine sont mélangés sous agitation pendant 10 minutes. 1,18 mL d'acide acétique sont ajoutés et le mélange est agité à température ambiante pendant 20 minutes. 5 g de diméthanesulfonate de 1,1'-(disulfanediyldiéthane-2,1-diyl)bis(4-méthylpyridinium) en suspension dans 7 mL d'iPrOH et 2 mL de méthanol sont additionnés. Le mélange réactionnel est maintenu agité pendant 24 h à température ambiante. Le précipité obtenu est filtré, lavé avec 100 mL d'acétone puis séché. 8,56 g de poudre sont recueillis. Les analyses indiquent que le produit est conforme et pur. RMN $^1H$ (400 MHz, MeOH-d4) 2.71 (s, 6 H), 3.35 (t, 4 H), 3.64 (t, 8 H), 3.76 (t, 8 H), 4.74 (t, 4 H), 6.83 (d, 4 H), 7.04 (d, 2 H), 7.58 (d, 4 H), 7.81 (d, 2 H), 7.96 (d, 4 H), 8.56 (d, 4 H).

<u>Exemple 2 :</u> **Synthèse du dibromure de 1,1'-(disulfanediyldiéthane-2,1-diyl)bis[4-((E)-2-{4-[éthyl(2-hydroxyéthyl)amino]-2-méthy)phényl}-1-méthylvinyl)pyridinium] [2]**

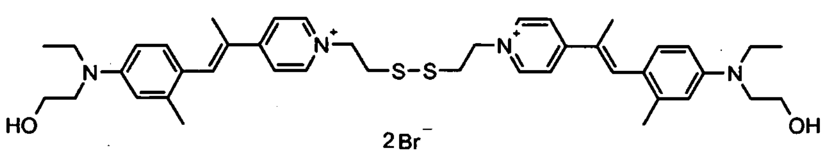

**[2]**

**Schéma de synthèse**

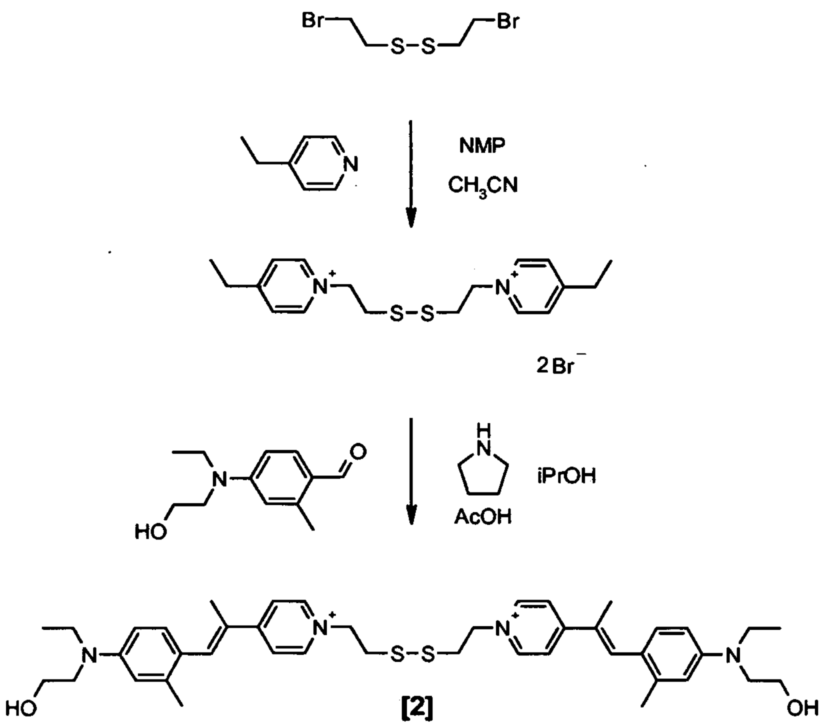


[2]

2 Br⁻

**Mode opératoire**

**Etape 1 : Synthèse du dibromure de 1,1'-(disulfanedlyldiéthane-2,1-diyl)bis(4-éthylpyridinium)**

12.,9 g de 4-éthyl-pyridine solubilisés dans 4 mL de NMP et 15 mL d'acétonitrile (ACN) sont agités et chauffés à 80 °C. 15 g de 1-Bromo-2-(2-bromo-éthyldisulfanyl)-éthane dilués dans 10 mL d'ACN sont ajoutés goutte à goutte au milieu pendant 30 minutes. Le mélange réactionnel est agité et chauffé à reflux pendant 4 h, puis à température ambiante pendant 17 h. Au solide vert obtenu, sont ajoutés 200 mL d'acétone, le solide est cassé, puis filtré, lavé avec 50 mL d'acétone, puis séché. 23,32 g de poudre verte sont recueillis. Les analyses indiquent que le produit est conforme.

**Etape 2 : Synthèse du dibromure de 1,1'-(disulfanediyldiéthane-2,1-diyl)bis[4-((E)-2-{4-(éthyl(2-hydroxyéthyl) amino]-2-méthylphényl}-1-méthylvinyl)pyridinium] [2]**

5 g de 4-[éthyl-(2-hydroxy-éthyl)-amino]-2-méthyl-benzaldéhyde, 10 mL d'iPrOH, et 1,74 mL de pyrrolidine sont mélangés sous agitation pendant 10 min. 1,21 mL d'acide acétique sont ajoutés et le mélange est agité à température ambiante pendant 20 min. 5 g de dibromure de 1,1'-(disulfanediyldiéthane-2,1-diyl)bis(4-éthylpyridinium) en suspension dans 7 mL d'iPrOH et 5 mL de méthanol sont additionnés. Le mélange réactionnel est maintenu agité pendant 1 semaine à température ambiante. Le mélange réactionnel est conservé à température ambiante pendant 4 semaines. Le mélange réactionnel est concentré, le résidu est extrait par un mélange $H_2O/CH_2Cl_2$, puis le produit attendu est extrait par du butanol, la phase organique est concentrée, et le résidu est repris par de l'acétate d'éthyle. Le précipité obtenu est filtré puis séché. 810 mg de poudre sont recueillis. Les analyses indiquent que le produit est conforme.

**Exemple 3 : Synthèse du diméthane sulfonate de 1,1'-(disulfanediyldiéthane-2,1-diyl)bis[4-((E)-2-{4-[[3-(diméthylamino)propyl](méthyl)amino]phényl}vinyl)-pyridinium] [3]**

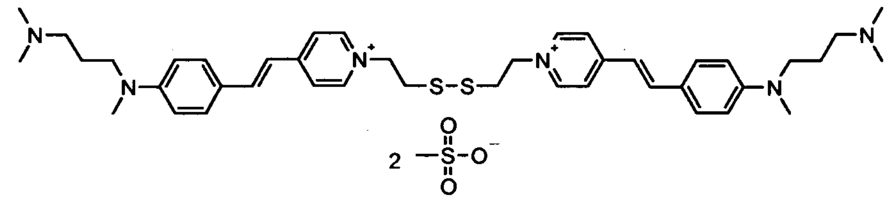

[3]

**Schéma de synthèse**

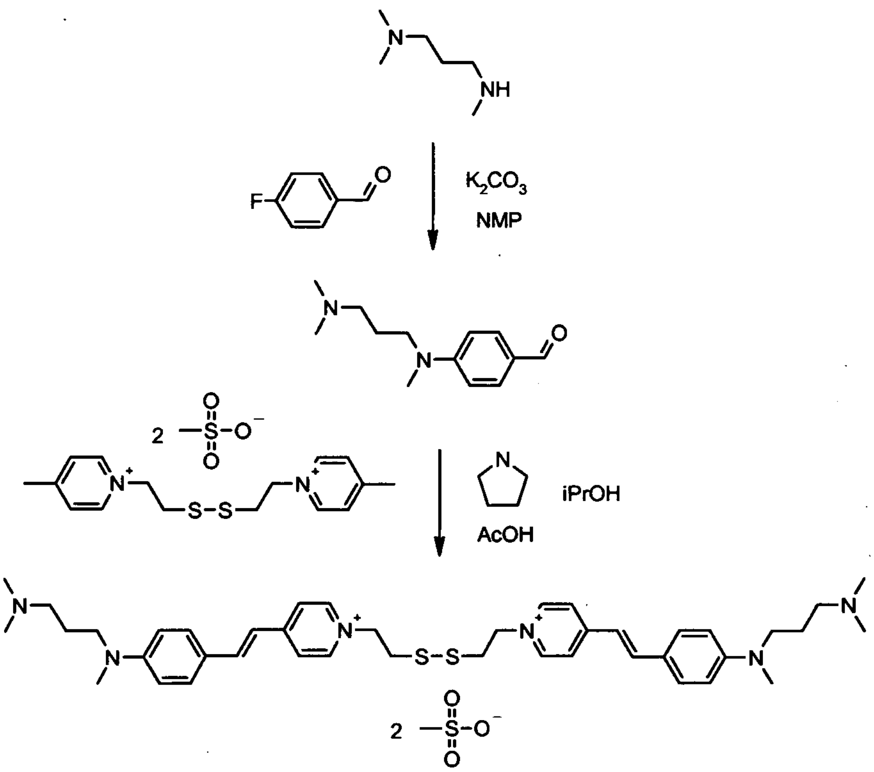


[3]

**Mode opératoire**

**Etape 1 : Synthèse du 4-[[3-(diméthylamino)propyl](méthyl)amino]benzaldéhyde**

10 g de 4-fluorobenzaldéhyde, 12 g de $K_2CO_3$, 20 mL de NMP sont agités et chauffés à 50 °C. 13 mL de N.N.N'-triméthyl-1.3-propanediamine sont additionnés goutte à goutte au milieu maintenu sous agitation et chauffé à 80 °C pendant 10 h. Après refroidissement du milieu réactionnel à température ambiante, 100 mL d'acétone sont ajoutés. Le précipité obtenu est filtré, le filtrat est évaporé à sec puis séché. 21.85 g de poudre sont recueillis. Les analyses indiquent que le produit est conforme.

**Etape 2: Synthèse du di méthane sulfonate de 1,1'-(disulfanediyldiéthane-2,1-diyl)bis[4-((E)-2-{4-[[3-(diméthylamino)propyl](méthyl)amino]phényl}vinyl)-pyridinium] [3]**

2 g de 4-[(3-diméthylamino-propyl)-méthyl-amino]-benzaldéhyde solubilisés dans 5 mL d'iPrOH, auxquels sont

ajoutés 585 μL de pyrrolidine sont agités à température ambiante pendant 10 minutes. 408 μL d'acide acétique sont additionnés au milieu réactionnel maintenu sous agitation à température ambiante pendant 20 minutes. 1,71 g de di methanesulfonate de 1,1'-(disulfanediyldiéthane-2,1-diyl)bis(4-méthyl-pyridinium) préalablement solubilisés dans 3 mL d'iPrOH et 1 mL de méthanol sont introduits dans le milieu agités à 20 °C pendant 4 jours. 50 mL d'éther diisopropyle sont ajoutés au mélange réactionnel refroidi par l'intermédiaire d'un bain de carboglace et d'acétone. Le précipité obtenu est filtré puis séchée. 2,21 g de poudre violette sont recueillis. Les analyses indiquent que le produit est conforme. RMN $^1$H (400 MHz, MeOH-d4) 1.84 (m 4 H), 2.34 (s, 12 H), 2.48 (t, 4H), 2.71 (s, 6 H), 3.06 (s, 6H), 3.36 (t, 4H), 3.50 (t, 4 H), 4.75 (t, 4 H), 6.79 (d, 4 H), 7.07 (d, 2 H), 7.60 (d, 4 H), 7.84 (d, 2 H), 7.98 (d, 4 H), 8.57 (d, 4 H).

**Exemple 4 : Synthèse du sel de 1,1'-(disulfanediyldiéthane-2,1-diyl)bis{2-[(E)-2-(4-{méthyl[3-(triméthylammonio)propyl]amino}phényl)vinyl]pyridinium} [4]**

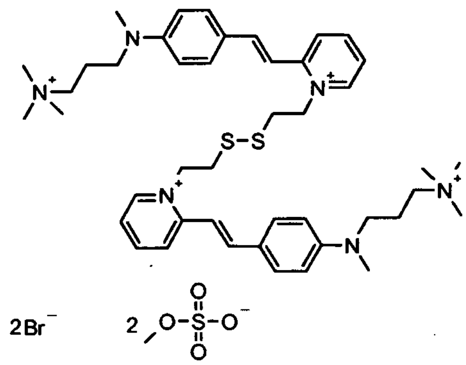

**[4]**

**Schéma de synthèse**

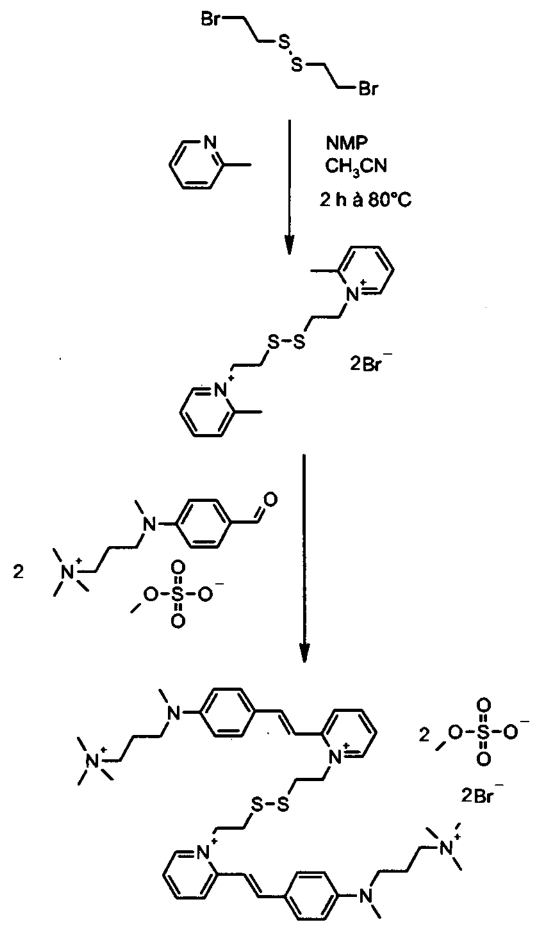


**[4]**

**Mode opératoire**

**Etape 1 : Synthèse du dibromure de 1,1'-(disulfanediyldiéthane-2,1-diyl)bis(2-méthyl-pyridinium)**

Un mélange de 56 g de 1-bromo-2-[(2-bromoéthyl)disulfanyl]éthane et de 15 mL de *N*-Méthylpyrrolidone (NMP) est versé goutte à goutte sur 35 g de 2-picoline sous agitation, à 80 °C. Le mélange (suspension blanche) est maintenu agité 30 min à 80 °C, 100 mL d'ACN sont ajoutés, l'agitation est maintenue à 80 °C pendant 90 min. Après refroidissement, le solide obtenu est filtré, lavé par 100 mL d'ACN puis séché. On recueille 56,2 g de poudre brune. 45 g de cette poudre sont mis en suspension dans 300 mL d'iPrOH, à reflux. Une fois la température baissée à 40 °C, le solide est filtré, lavé par 3 fois 100 mL d'iPrOH et séché sous vide. Produit beige clair, 40,56 g. Analyses en conformité avec la structure attendue.

**Etape 2 : Synthèse du méthylsulfate de 3-[(4-formylphényl)(méthyl)amino]-N,N,N-triméthylpropan-1-aminium**

5 g de -[[3-(diméthylamino)propyl](méthyl)amino]benzaldéhyde sont dilués dans 15 mL de dichlorométhane. 2,36 mL de diméthylsulfate dilués dans 10 mL de dichlorométhane sont progressivement ajoutés. Le mélange est chauffé à reflux pendant 4 h puis refroidi à température ambiante et versé sur 100 mL d'acétate d'éthyle. L'huile brune formée est décantée, lavée par 100 mL d'AcOEt, reprise dans 200 mL de méthanol et concentrée sous vide. Elle est ensuite dissoute dans une solution saturée de bicarbonate de soude et de chlorure de sodium et extraite au butanol. La phase butanol est concentrée sous vide, séché sous vide en présence de $P_2O_5$, puis le produit est utilisé pour l'étape suivante.

**Etape 3 : Synthèse du sel de 1,1'-(disulfanediyldiéthane-2,1-diyl)bis{2-[(E)-2-(4-{méthyl[3-(triméthylammonio) propyl]amino}phényl)vinyl]pyridinium} [4]**

0,62 g de sulfate de 3-[(4-formylphényl)(méthyl)amino]-N,N,N-triméthylpropan-1-aminium méthyl solubilisés

dans 2 mL d'iPrOH et 3 mL de méthanol auxquels sont ajoutés 0,15 mL de pyrrolidine sont agités à température ambiante pendant 10 minutes. 0,11 mL d'acide acétique sont additionnés au milieu réactionnel maintenu sous agitation à température ambiante pendant 20 minutes. 0,41 g de dibromure de 1,1'-(disulfanediyldiéthane-2,1-diyl)bis(2-méthyl-pyridinium) préalablement solubilisés dans 2 mL d'iPrOH et 2 mL de méthanol sont introduits dans le milieu agités à 20 °C pendant 17 h. Les analyses montrent la présence du produit attendue.

**Exemple 5 : Synthèse du méthanesulfonate de 4-((E)-2-{4-[bis(2-hydroxyéthyl)amino]-phényl}vinyl)-1-(2-sulfanyléthyl)pyridinium [5]**

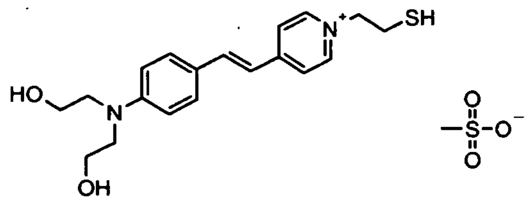

**[5]**

**Schéma de synthèse**

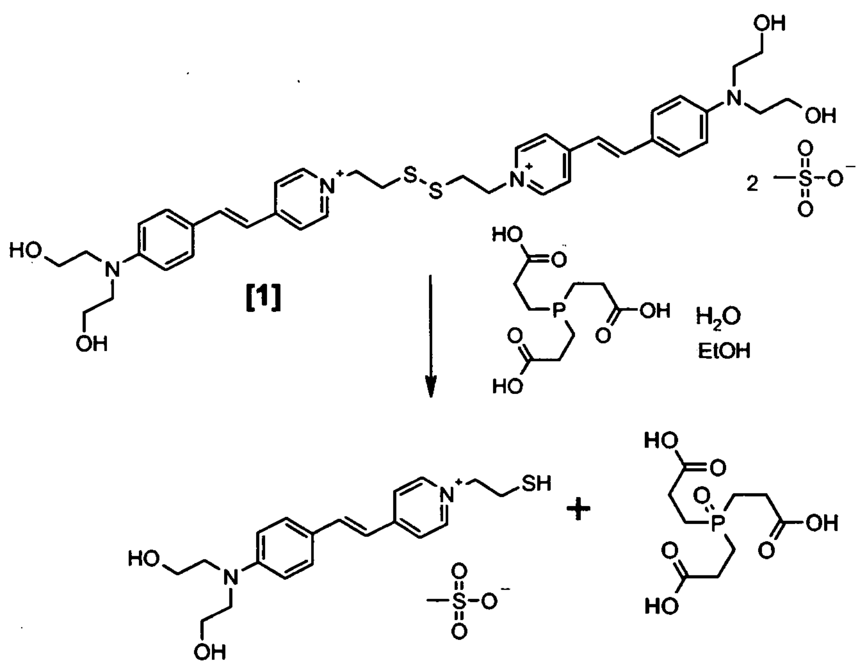


**[5]**

**Mode opératoire**

**Synthèse du sel de 4-((E)-2-{4-[bis(2-hydroxyé éthyl)amino]phényl}-vinyl)-1-(2-sulfanyléthyl)pyridinium [5]**

88 mg du composé [1] sont dissous dans 10 mL de mélange eau/éthanol (1/1); 60 mg (2 éq.) d'hydrate de chlorhydrate d'acide 3-[bis(2-carboxy-éthyl)phosphino]propanoïque en solution dans 1 mL d'eau et 21 mg (4 éq) de bicarbonate de sodium en solution dans 1 mL d'eau sont ajoutés au mélange. Après 30 minutes d'agitation à 40°C sous

atmosphère inerte, les analyses indiquent que le mélange contient très majoritairement le produit attendu [5].

Analyse LC-MS : LC-DAD (400-700 nm)
Colonne : Waters XTerra MS C18 5µm (4.6 x 50) mm
Phase Mobile : A : Eau + acide formique 0. 1 % / B : acétonitrile
Gradient linéaire : T (min) A%/B% : 0 min 95/5 ; 8 min 0/100
Débit: 1 mL/min.
Détection : UV Barrette de diodes λ=400-700nm

Temps de rétention t = 3.3 min
Pureté relative >90%
MS (ESI+) m/z=345 correspond au pic de masse du monocation du produit attendu [5]

**EXEMPLES DE COLORATION**

**Exemple 1 - procédé de coloration avec les composés [1] à [4]**

Préparation d'une composition A

| | |
|---|---|
| Colorant disulfure de [1] à [4] | $10^{-3}$ mol% |
| Alcool Benzylique | 4 g |
| Polyéthylèneglycol 6OE | 6 g |
| Hydroxyethylcellulose | 0,7 g |
| Alkylpolyglucoside en solution aqueuse à 65%MA | 4.5 g |
| Eau déminéralisée | qsp 100g |

Préparation d'une composition B

| | |
|---|---|
| Acide thioglycolique | 1 M |
| Hydroxyde de Sodium | qsp pH 8,5 |
| Eau déminéralisée | qsp 100g |

Au moment de l'emploi, on mélange les compositions A (9 mL) et B (1 mL), puis on applique le mélange obtenu sur une mèche de 1 g de cheveux foncés (hauteur de ton 4) pendant 30 minutes à température ambiante(les mèches sont retournées et réimprégnées après 15 minutes).

Après rinçage à l'eau courante et séchage, on observe un éclaircissement des cheveux ainsi traités : la mèche de hauteur de ton 4 est devenue visuellement plus claire que des mèches témoins non traitées.

**Exemple 2 - procédé de coloration avec le composé [5]**

10 mL de solution fraîche du composé [5] de l'exemple de synthèse sont appliqués sur une mèche de 1 g cheveux de hauteur de ton 4 disposées au fond d'un même bol pendant 30 minutes à température ambiante (les mèches sont retournées et réimprégnées après 15 minutes).
Les mèches sont ensuite rincées à l'eau courante et séchées.
Après coloration la mèche de hauteur de ton 4 est devenue visuellement plus claire que des mèches témoins non traitées.

**Rémanence vis-à-vis des shampooings successifs :**

Les mèches ainsi traitées sont divisées en deux, une moitié est soumise à 5 shampooings successifs selon

un cycle qui comprend le mouillage des mèches à l'eau, le lavage avec un shampooing classique, un rinçage à l'eau suivi d'un séchage.

*Observations visuelles*

Lors des shampooings, il n'y a pas de dégorgement visible, la mousse des shampooings et les eaux de rinçage ne sont pas colorées

La couleur observée sur les mèches est conservée et l'effet d'éclaircissement reste visible sur les cheveux ainsi traités

*Résultats dans le système L*a*b* :*

La couleur des mèches avant et après les 5 lavages a été évaluée dans le système L*a*b* au moyen d'un spectrophotomètre CM 2600D MINOLTA ®, (Illuminant D65).

Dans ce système L*a*b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*,a*et b* représentent les valeurs mesurées avant coloration et $L_0$*, $a_0$* et $b_0$* représentent les valeurs mesurées avant coloration (ou shampooing).

Plus la valeur de ΔE est importante, plus la différence de couleur entre les mèches HT4 et les mèches colorées est importante.

| Traitement avec le colorant fluorescent sur les mèches de HT4 | ΔE |
|---|---|
| | |
| Après application du composé 1 selon l'invention | 8,38 |
| Après application du composé 1 selon l'invention et après 5 shampooings successifs | 8,66 |
| | |
| Après application du composé 2 selon l'invention | 2,71 |
| Après application du composé 2 selon l'invention et après 5 shampooings successifs | 2,84 |
| | |
| Après application du composé 3 selon l'invention | 9,62 |
| Après application du composé 3 selon l'invention et après 5 shampooings successifs | 9,88 |
| | |
| Après application du composé 5 selon l'invention | 5,27 |
| Après application du composé 5 selon l'invention et après 5 shampooings successifs | 5,25 |

Les résultats du tableau ci-dessus montrent que la coloration évolue très peu après 5 shampooings. Ainsi la coloration et l'effet éclaircissant sur les cheveux restent quasiment inchangés ce qui montre une très bonne résistance aux shampooings des colorants de l'invention.

*Résultats de Réflectance :*

Les performances d'éclaircissement des compositions conformes à l'invention et leur rémanence vis-à-vis de shampooings successifs ont été exprimées en fonction de la réflectance des cheveux. Ces réflectances sont comparées à la réflectance d'une mèche de cheveux non traitées de hauteur de ton HT4 .

La réflectance est mesurée au moyen d'un appareil spectrophotocolorimètre KONIKA-MINOLTA®, CM 2600d et après irradiation des cheveux avec une lumière visible dans la gamme de longueurs d'onde allant de 400 à 700 nanomètres.

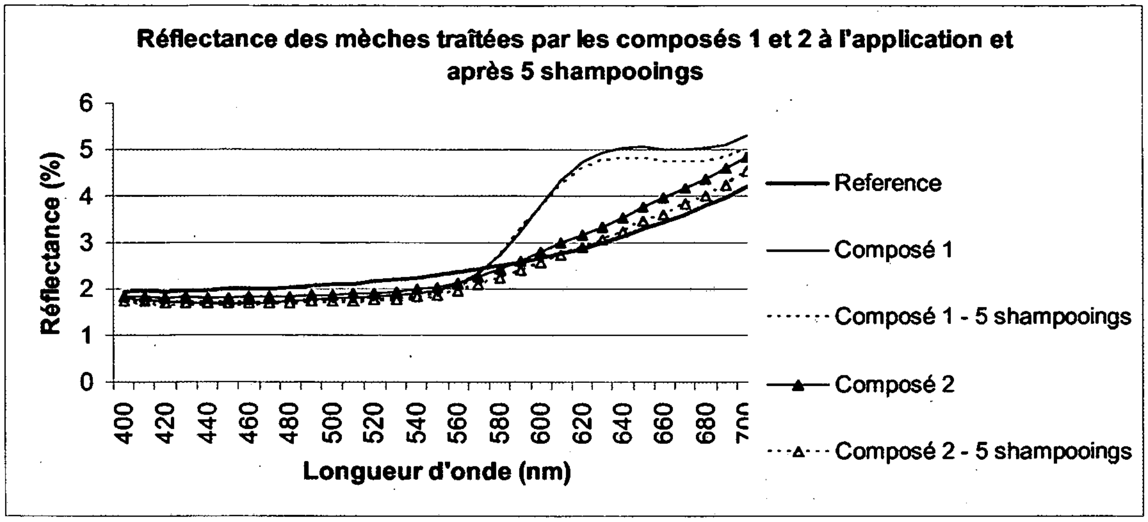


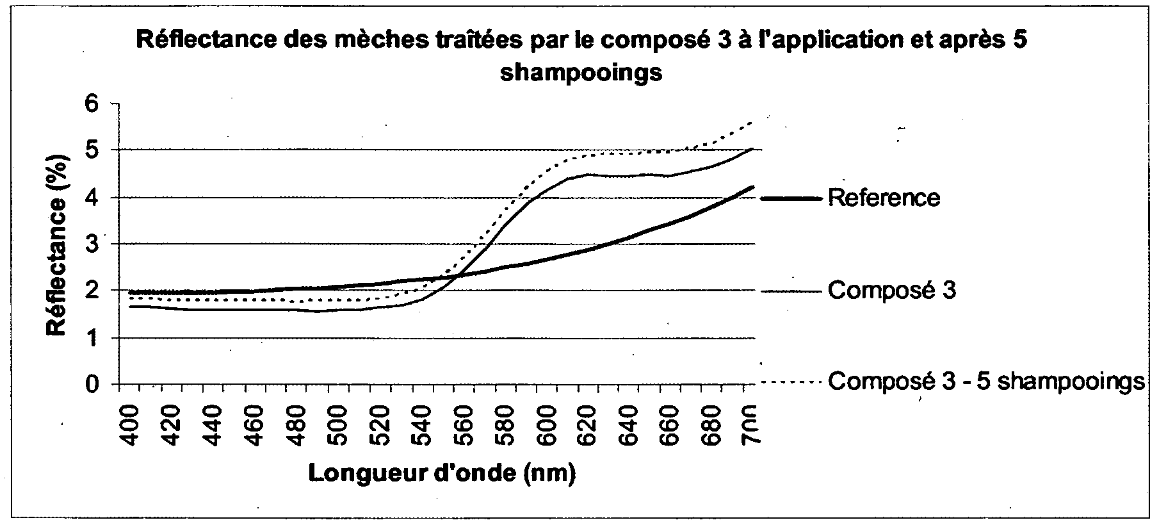

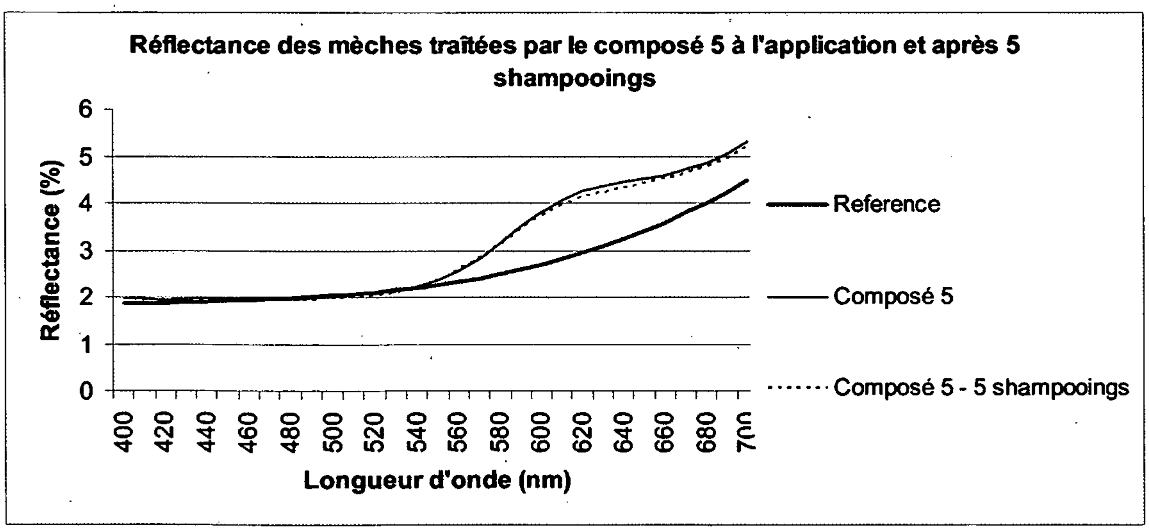


On constate tout d'abord que la réflectance d'une mèche de cheveux traitée avec une composition selon l'invention est supérieure à celle des cheveux non traités. Les mèches traitées apparaissent donc plus claires.

De plus, les résultats montrent que la réflectance des mèches de cheveux de hauteur de ton 4 traitées avec les compositions de l'invention évoluent très peu après 5 shampooings. Ainsi la coloration et l'effet éclaircissant sur les cheveux restent quasiment inchangés ce qui montre une très bonne résistance aux shampooings des colorants de l'invention.

## Revendications

1. Colorant fluorescent thiol de formule (I) ou (II) :

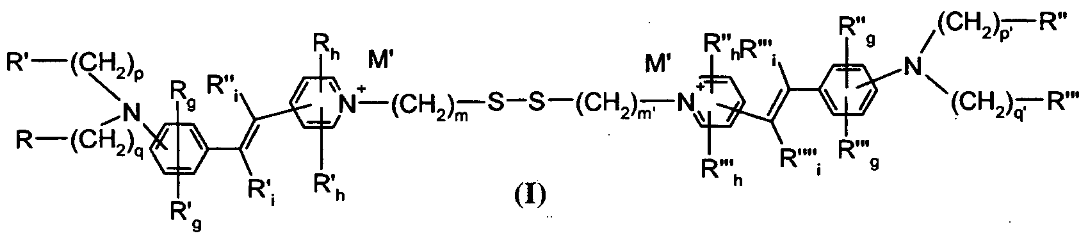

(I)

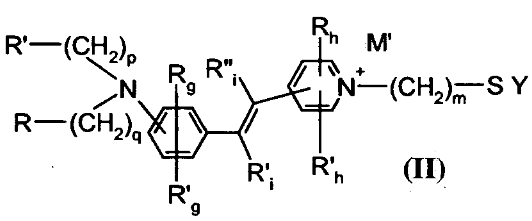

(II)

ses sels d'acide organique ou minéral, isomères optiques, isomères géométriques, et les solvates tels que hydrates ; formules (I) et (II) dans lesquelles :

➢ R et R''', identiques ou différents, représentent un groupement hydroxy, un groupement amino ($NR_aR_b$) ou ammonium ($N^+R_aR_bR_c$), $An^-$ ; avec $R_a$, $R_b$ et $R_c$, identiques ou différents, représentant un atome d'hydrogène ou un groupement $(C_1\text{-}C_4)$alkyle ; ou alors deux groupes alkyle $R_a$ et $R_b$ du groupement amino ou ammonium forment un hétérocycle comprenant de 5 à 7 chaînons et comprenant éventuellement un autre hétéroatome identique ou différent à celui de l'atome d'azote et $An^-$ représentant un contre-ion anionique ;

➢ R' et R'', identiques ou différents, représentent un atome d'hydrogène ou un groupement tel que défini pour

R et R"' respectivement ;

➢ $R_g$, $R'_g$, $R''_g$, $R'''_g$, $R_h$, $R'_h$, $R''_h$ et $R'''_h$, identiques ou différents, représentent un atome d'hydrogène, d'halogène, un groupement amino, (di)($C_1$-$C_4$)alkylamino, cyano, carboxy, hydroxy, trifluorométhyle, acylamino, alcoxy en $C_1$-$C_4$, (poly)hydroxyalcoxy en $C_2$-$C_4$, ($C_1$-$C_4$)alkylcarbonyloxy, ($C_1$-$C_4$)alcoxycarbonyle, ($C_1$-$C_4$)alkylcarbonylamino, acylamino, carbamoyle , ($C_1$-$C_4$)alkylsulfonylamino, un radical aminosulfonyle, ou un radical ($C_1$-$C_{16}$) alkyle éventuellement substitué par un groupement choisi parmi ($C_1$-$C_{12}$)alcoxy, hydroxy, cyano, carboxy, amino, (di)($C_1$-$C_4$)alkylamino, ou alors les deux radicaux alkyle portés par l'atome d'azote du groupement amino forment un hétérocycle comprenant de 5 à 7 chaînons et comprenant éventuellement un autre hétéroatome identique ou différent à celui de l'atome d'azote ;

➢ $R'_i$, $R''_i$, $R'''_i$ et $R''''_i$, identiques ou différents, représentent un atome d'hydrogène, ou un groupement ($C_1$-$C_4$) alkyle ;

➢ m, m', identiques ou différents, représentent un entier compris inclusivement entre 1 et 10 ;

➢ p, p', q et q', identiques ou différents, représentent un entier compris inclusivement entre 1 et 6 ;

➢ M' représentant un contre-ion anionique ; et

➢ Y représente : i) un atome d'hydrogène ; ii) un métal alcalin ; iii) un métal alcalino-terreux; iv) un groupement ammonium: $N^+R^\alpha R^\beta R^\gamma R^\delta$ ou un groupement phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe ($C_1$-$C_4$)alkyle ; ou v) un groupement protecteur de fonction thiol

étant entendu que :

- lorsque le composé de formule (1) ou (II) contient d'autres parties cationiques, il se trouve associé à un ou plusieurs contre-ions anioniques permettant d'atteindre l'électroneutralité de la formule (I) ou (II).

**2.** Colorant fluorescent de formule (II) selon la revendication précédente dans lequel Y représente un atome d'hydrogène, ou un métal alcalin.

**3.** Colorant fluorescent de formule (II) selon la revendication 1 dans lequel Y représente un groupement protecteur.

**4.** Colorant fluorescent de formule (II) selon la revendication précédente dans lequel Y représente un groupement protecteur choisi parmi les radicaux suivants :

• ($C_1$-$C_4$)alkylcarbonyle ;
• ($C_1$-$C_4$)alkylthiocarbonyle ;
• ($C_1$-$C_4$)alcoxycarbonyle ;
• ($C_1$-$C_4$)alcoxythiocarbonyle ;
• ($C_1$-$C_4$)alkylthio-thiocarbonyle ;
• (di) ($C_1$-$C_4$) (alkyl)aminocarbonyle ;
• (di) ($C_1$-$C_4$) (alkyl)aminothiocarbonyle;
• arylcarbonyle comme phénylcarbonyle ;
• aryloxycarbonyle ;
• aryl($C_1$-$C_4$)alkcoxycarbonyle ;
• (di) ($C_1$-$C_4$) (alkyl)aminocarbonyle ;
• ($C_1$-$C_4$)(alkyl)arylaminocarbonyle ;
• carboxy ;
• $SO_3^-$ , $M^+$ avec $M^+$ représentant un métal alcalin ou alors M' de la formule (II) et $M^+$ sont absents ;
• aryle éventuellement substitué ;
• hétéroaryle éventuellement substitué ;
• hétérocycloalkyle éventuellement substitué, éventuellement cationique ;
• isothiouronium -C($NR'^cR'^d$)=$N^+R'^eR'^f$; $An^-$ avec $R'^c$, $R'^d$, $R'^e$ et $R'^f$, identiques ou différents représentent un atome d'hydrogène ou un groupement ($C_1$-$C_4$)alkyle ; préférentiellement $R'^c$ à $R'^f$ représentent un atome d'hydrogène ; et $An^-$ représente un contre-ion ;
• isothiourée -C($NR'^cR'^d$)=$NR'^e$; avec $R'^c$, $R'^d$, et $R'^e$ tels que définis précédemment ;
• (di)aryl($C_1$-$C_4$)alkyle éventuellement substitué ;
• (di)hétéroaryl($C_1$-$C_4$)akyle éventuellement substitué ;
• $CR^1R^2R^3$ avec $R^1$, $R^2$ et $R^3$ identiques ou différents, représentant un atome d'halogène ou un groupe choisi parmi :

- $(C_1-C_4)$ alkyle ;
- $(C_1-C_4)$ alcoxy ;
- aryle éventuellement substitué ;
- hétéroaryle éventuellement substitué ;
- $P(Z^1)R'^1R'^2R'^3$ avec $R'^1$, et $R'^2$ identiques ou différents représentent un groupement hydroxy, $(C_1-C_4)$ alcoxy ou alkyle, $R'^3$ représente un groupement hydroxy ou $(C_1-C_4)$alcoxy, et $Z^1$ représente un atome d'oxygène ou de soufre ;

• cyclique stériquement encombré tel que le groupe adamantyle ; et
• alcoxy$(C_1-C_4)$alkyle éventuellement substitué.

**5.** Colorant fluorescent selon l'une quelconque des revendications précédentes dans lequel Y représente un métal alcalin ou un groupement protecteur choisi parmi :

➢ $(C_1-C_4)$alkylcarbonyle ;
➢ arylcarbonyle ;
➢ $(C_1-C_4)$alcoxycarbonyle ;
➢aryloxycarbonyle ;
➢ aryl$(C_1-C_4)$alcoxycarbonyle ;
➢ (di) $(C_1-C_4)$ (alkyl)aminocarbonyle ;
➢ $(C_1-C_4)$(alkyl)arylaminocarbonyle ;
➢ aryle éventuellement substitué;
➢ hétéroaryle monocyclique cationique à 5, 6 chaînons ;
➢ hétéroaryle bicyclique cationique à 8 à 11 chaînons ;
➢ hétérocycle cationique de formule suivante :

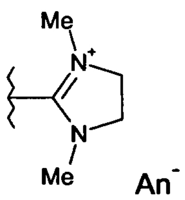

➢ isothiouronium $-C(NH_2)=N^+H_2$; $An^-$;
➢ isothiourée $-C(NH_2)=NH$ ; et
➢ $SO_3^-$, $M^+$ avec $M^+$ représentant un métal alcalin ou alors M' de la formule (II) et $M^+$ sont absents.

**6.** Colorant fluorescent selon l'une quelconque des revendications précédentes appartenant à la formule (Ia) ou (IIa) qui possède le groupement éthylène reliant la partie pyridinium au phényle en position ortho ou para du pyridinium soit en 2-4', 4-2', 4-4':

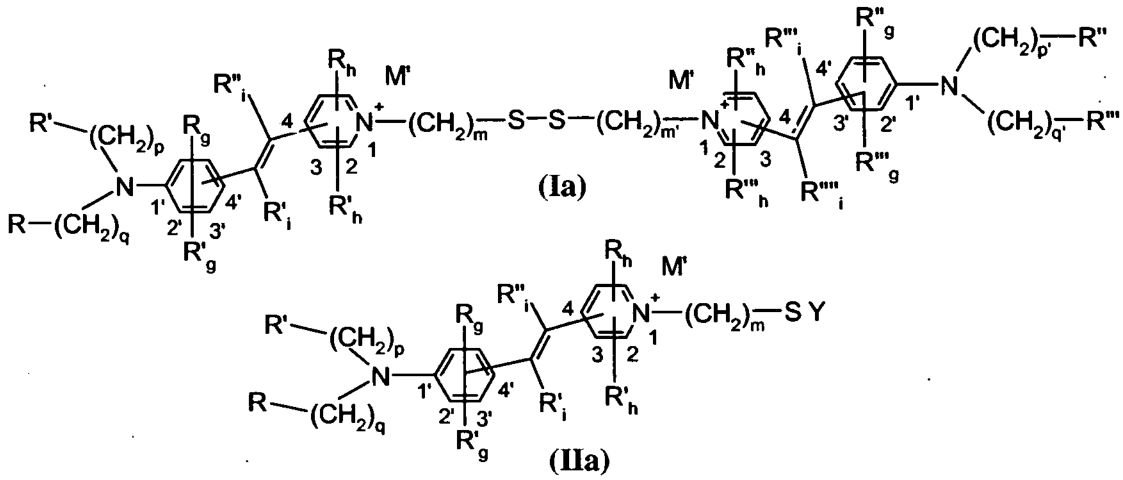

(Ia)

(IIa)

avec R, R', R", R"', $R_g$, $R'_g$, $R''_g$, $R''_g$, $R_h$, $R'_h$, $R''_h$, $R'''_h$, $R'_i$, $R''_i$, $R'''_i$, $R''''_i$, m, m', p, p', q, q', Y et M' sont tels que définis dans une quelconque des revendications précédentes.

**7.** Colorant fluorescent selon l'une quelconque des revendications précédentes, choisi parmi les colorants suivants :

| | |
|---|---|
| 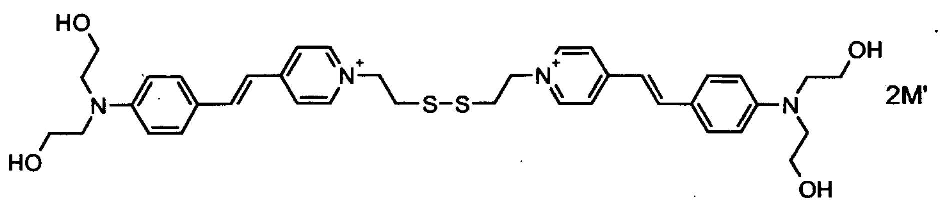 2M' | |
| 1 | |
| 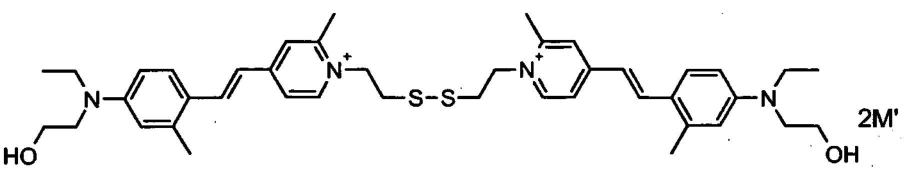 2M' | |
| 2 | |
| 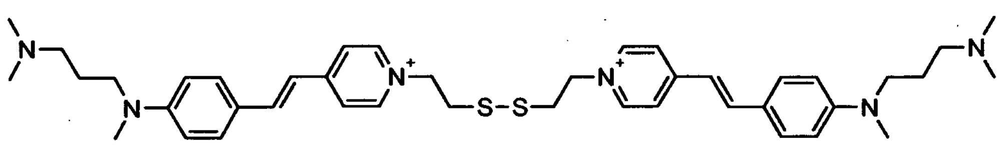 2M' | |
| 3 | |
| 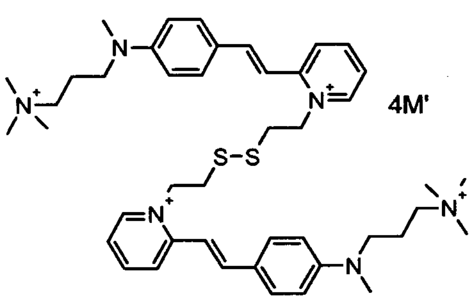 4M' | 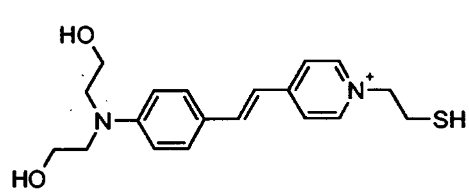 M' |
| 4 | 5 |

(suite)

| | |
|---|---|
| 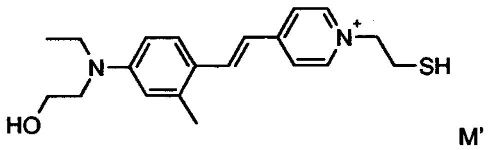 | 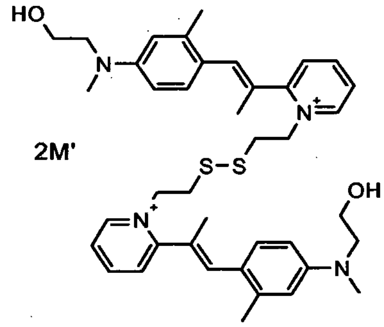 |
| **6** | **7** |
| 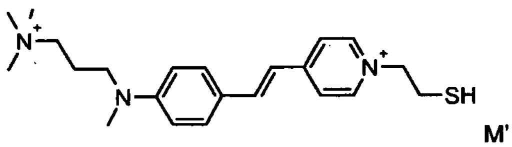 | 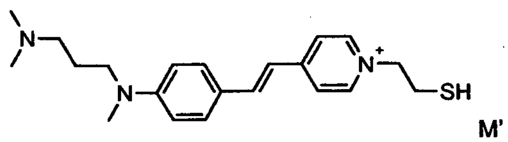 |
| **8** | **9** |
| 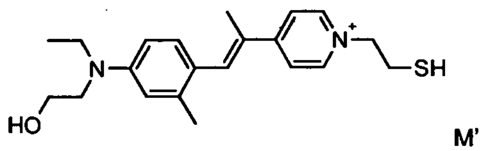 | 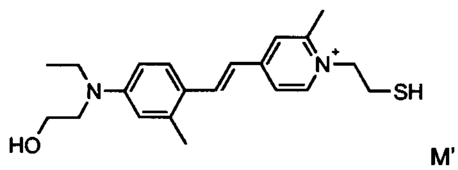 |
| **10** | **11** |
| 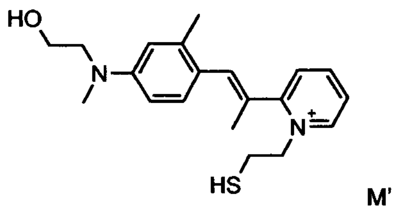 | 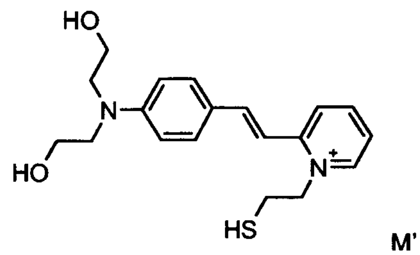 |
| **12** | **13** |
| 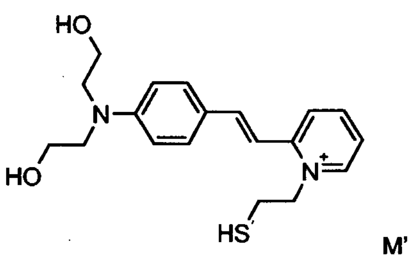 | |
| **14** | |

(suite)

| | |
|---|---|
| 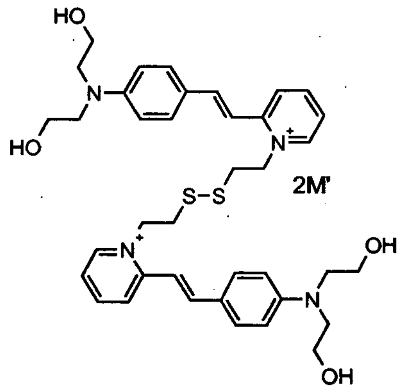 2M' | 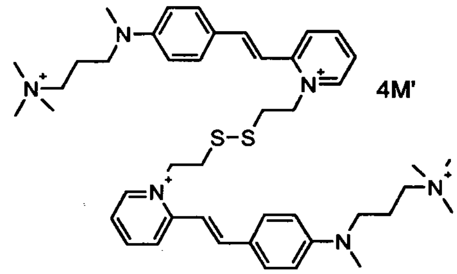 4M' |
| **16** | **17** |
| 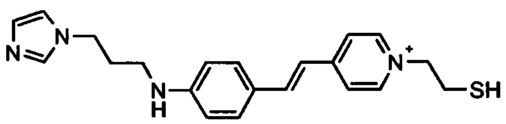 M' | 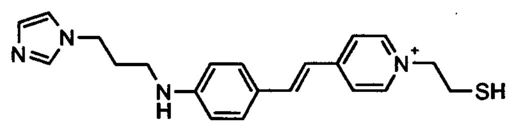 M' |
| **18** | **19** |
| 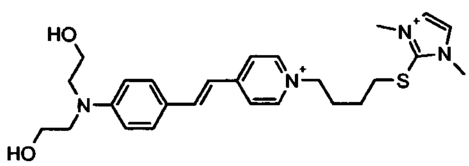 2M' | 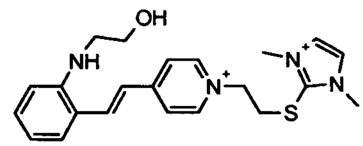 2M' |
| **20** | **21** |
| 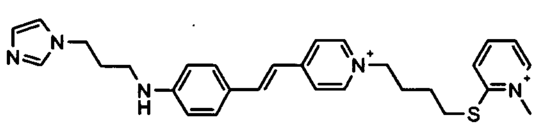 2M' | 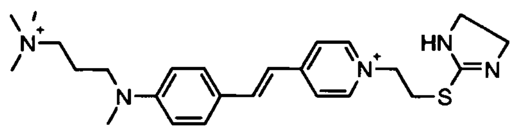 2M' |
| **22** | **23** |
| 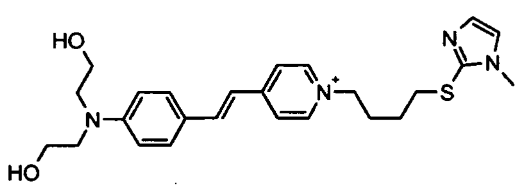 M' | 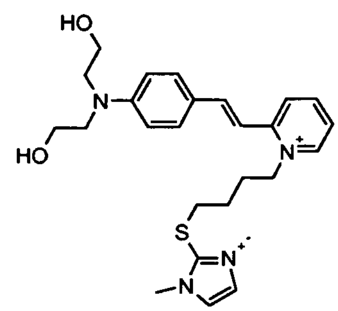 2M' |
| **24** | **25** |
| 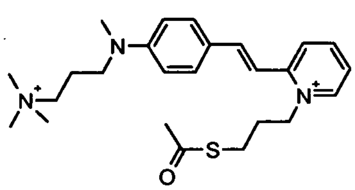 2M' | 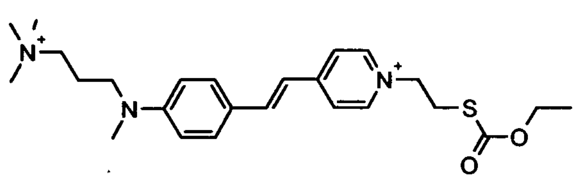 |

(suite)

| 26 | 27 |
|---|---|
| 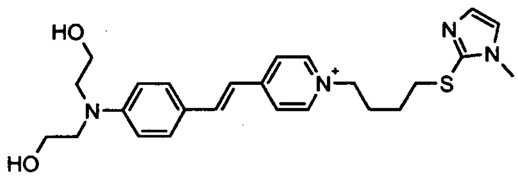 M' | 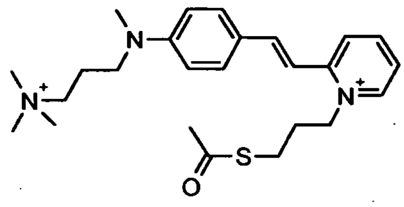 2M' |
| 28 | 29 |
| 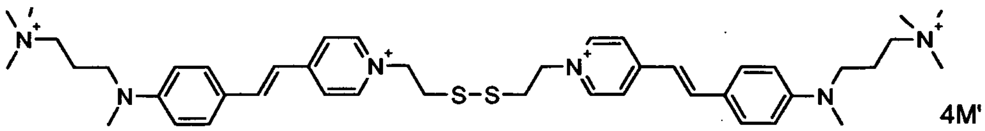 4M' | |
| 30 | |
| 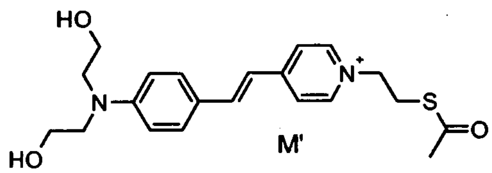 M' | |
| 31 | |
| 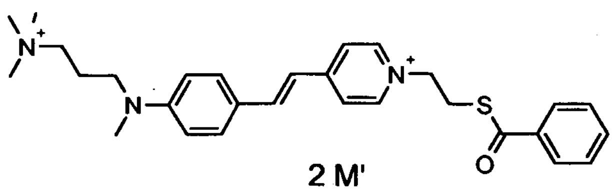 2 M' | |
| 32 | |

avec M' représentant un contre-ion anionique.

**8.** Composition tinctoriale comprenant dans un milieu cosmétique approprié, un colorant fluorescent de formule (I) ou (II) tel que défini dans une quelconque des revendications 1 à 7.

**9.** Composition tinctoriale comprenant dans un milieu cosmétique approprié, au moins un colorant fluorescent de formule (I) ou (II) tel que défini dans une quelconque des revendications 1 à 7 et au moins un agent réducteur.

**10.** Composition tinctoriale selon la revendication précédente dans laquelle l'agent réducteur est choisi parmi, la cystéine, l'homocystéine, l'acide thiolactique, les sels de ces thiols, les phosphines, le bisulfite, les sulfites, l'acide thioglycolique, ainsi que ses esters, les borohydrures et leurs dérivés, les sels de sodium, lithium, potassium, calcium, ammoniums quaternaires ; le catécholborane.

**11.** Composition selon l'une quelconque des revendications 8 à 10, dans laquelle le colorant fluorescent de formule (I) ou (II) est présent en quantité comprise entre 0,001 et 50% en poids par rapport au poids total de la composition.

**12.** Procédé de coloration de matières kératiniques, dans lequel on applique sur les matières, une composition tinctoriale telle que définie dans une quelconque des revendications 8 à 11, comprenant dans un milieu cosmétique approprié, au moins un colorant fluorescent de formule (1) ou (II) tel que défini dans une quelconque des revendications 1 à 7, éventuellement en présence d'un agent réducteur.

**13.** Procédé de coloration selon la revendication précédente dans lequel lorsque le colorant fluorescent thiol de formule (II) comprend un groupement Y de protection, l'application est précédée d'une étape de déprotection.

**14.** Procédé de coloration de matières kératiniques selon l'une quelconque des revendications 12 ou 13 **caractérisé en ce que** les matières kératiniques sont des fibres kératiniques foncées possédant une hauteur de ton inférieure ou égale à 6.

**15.** Procédé selon l'une quelconque des revendications 12 à 14 dans lequel l'agent réducteur est appliqué avant ou après l'application du colorant fluorescent de formule (I) ou (II).

**16.** Procédé selon une quelconque des revendications 12 à 15, dans lequel la composition comprend un agent oxydant.

**17.** Procédé selon l'une quelconque des revendications 12 à 16 comprenant une étape supplémentaire consistant à appliquer sur les fibres kératiniques un agent oxydant.

**18.** Dispositif à plusieurs compartiments dans lequel un premier compartiment contient une composition tinctoriale comprenant un colorant fluorescent tel de formule (1) ou (II) que défini aux revendications 1 à 7 et un deuxième compartiment contient un agent réducteur.

**19.** Dispositif selon la revendication précédente comprenant un troisième compartiment qui contient un agent oxydant.

**20.** Utilisation des colorants fluorescents de formule (I) ou (II) tels que définis aux revendications 1 à 7 pour la teinture de fibres kératiniques humaines foncées.

**21.** Utilisation selon la revendication précédente pour l'éclaircissement des fibres kératiniques foncées.

**22.** Utilisation selon les revendications 20 ou 21 **caractérisée en ce que** les fibres kératiniques possèdent une hauteur de ton inférieure à 6.

## Claims

**1.** Thiol fluorescent dye of formula (I) or (II):

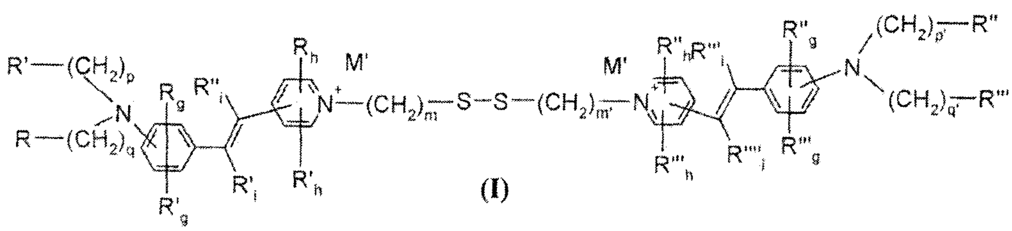

(I)

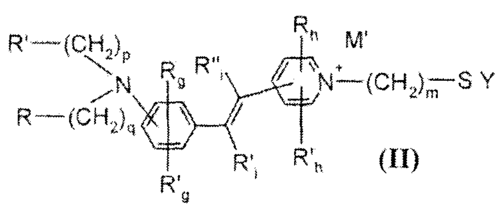

(II)

the organic or mineral acid salts, optical isomers and geometrical isomers thereof, and the solvates such as hydrates; in which formulae (I) and (II):

➢ R and R''', which may be identical or different, represent a hydroxyl group, an amino group ($NR_aR_b$) or an ammonium group ($N^+R_aR_bR_c$) , $An^-$; with $R_a$, $R_b$ and $R_c$, which may be identical or different, representing a

hydrogen atom or a ($C_1$-$C_4$) alkyl group; or else two alkyl groups $R_a$ and $R_b$ of the amino or ammonium group form a heterocycle comprising 5 to 7 members and optionally comprising another heteroatom which may be identical to or different from that of the nitrogen atom and $An^-$ representing an anionic counterion;

➢ R' and R", which may be identical or différent, represent a hydrogen atom or a group as defined for R and R'" respectively;

➢ $R_g$, $R'_g$, $R''_g$, $R'''_g$, $R_h$, $R'_h$, $R''_h$ and $R'''_h$, which may be identical or different, represent a hydrogen or halogen atom, an amino, (di) ($C_1$-$C_4$)alkylamino, cyano, carboxyl, hydroxyl, trifluoromethyl, acylamino, $C_1$-$C_4$ alkoxy, $C_2$-$C_4$ (poly)hydroxyalkoxy, ($C_1$-$C_4$)alkylcarbonyloxy, ($C_1$-$C_4$)alkoxycarbonyl, ($C_1$-$C_4$)alkylcarbonylamino, acylamino, carbamoyl or ($C_1$-$C_4$) alkylsulphonylamino group, an aminosulphonyl radical, or a ($C_1$-$C_{16}$)alkyl radical optionally substituted with a group chosen from ($C_1$-$C_{12}$)alkoxy , hydroxy, cyano, carboxyl, amino and (di) ($C_1$-$C_4$)alkylamino, or else the two alkyl radicals borne by the nitrogen atom of the amino group form a heterocycle comprising 5 to 7 members and optionally comprising another heteroatom which may be identical to or different from that of the nitrogen atom;

➢ $R'_i$, $R''_i$, $R'''_i$ and $R''''_i$, which may be identical or different, represent a hydrogen atom or a ($C_1$-$C_4$) alkyl group ;

➢ m and m', which may be identical or different, represent an integer between 1 and 10 inclusive;

➢ p, p' , q and q, which may be identical or different, represent an integer between 1 and 6 inclusive;

➢ M' represents an anionic counterion; and

➢ Y represents: i) a hydrogen atom; ii) an alkali métal; iii) an alkaline earth metal; iv) an ammonium group: $N^+R^\alpha R^\beta R^\gamma R^\delta$ or a phosphonium group: $P^+R^\alpha R^\beta R^\gamma R^\delta$ with $R^\alpha$, $R^\beta$, $R^\gamma$ and $R^\delta$, which may be identical or different, representing a hydrogen atom or a ($C_1$-$C_4$) alkyl group; or v) a thiol-function-protecting group;

it being understood that:

- when the compound of formula (I) or (II) contains other cationic parts, it is associated with one or more anionic counterions allowing formula (I) or (II) to achieve electroneutrality.

**2.** Fluorescent dye of formula (II) according to the preceding claim, in which Y represents a hydrogen atom or an alkali metal.

**3.** Fluorescent dye of formula (II) according to Claim 1, in which Y represents a protecting group.

**4.** Fluorescent dye of formula (II) according to the preceding claim, in which Y represents a protecting group chosen from the following radicals:

- ($C_1$-$C_4$)alkylcarbonyl;
- ($C_1$-$C_4$)alkylthiocarbonyl;
- ($C_1$-$C_4$) alkoxycarbonyl;
- ($C_1$-$C_4$)alkoxythiocarbonyl;
- ($C_1$-$C_4$)alkylthiothiocarbonyl;
- (di) ($C_1$-$C_4$) (alkyl)aminocarbonyl;
- (di) ($C_1$-$C_4$) (alkyl)aminothiocarbonyl;
- arylcarbonyl such as phenylcarbonyl;
- aryloxycarbonyl;
- aryl($C_1$-$C_4$)alkoxycarbonyl;
- (di) ($C_1$-$C_4$) (alkyl)aminocarbonyl;
- ($C_1$-$C_4$) (alkyl)arylaminocarbonyl;
- carboxyl;
- $SO_3^-$, $M^+$ with $M^+$ representing an alkali metal or else M' of formula (II) and $M^+$ are absent ;
- optionally substitituted aryl;
- optionally substituted heteroaryl;
- optionally cationic, optionally substituted heterocycloalkyl;
- isothiouronium $-C(NR'^cR'^d)=N^+R'^eR'^f$; $An^-$ with $R'^c$, $R'^d$ $R'^e$ and $R'^f$, which may be identical or different, representing a hydrogen atom or a ($C_1$-$C_4$)alkyl group; preferably, $R'^c$ to $R'^f$ represent a hydrogen atom; and $An^-$ represents a counterion;
- isothiourea $-C(NR'^cR'^d)=NR'^e$; with $R'^c$, $R'^d$ and $R'^e$ as defined above;
- optionally substituted (di)aryl($C_1$-$C_4$)alkyl;
- optionally substituted (di)heteroaryl($C_1$-$C_4$)alkyl;
- $CR^1R^2R^3$ with $R^1$, $R^2$ and $R^3$, which may be identical or different, representing a halogen atom or a group

chosen from:

- $(C_1-C_4)$ alkyl;
- $(C_1-C_4)$alkoxy;
- optionally substituted aryl;
- optionally substituted heteroaryl;
- $P(Z^1)R'^1R'^3$ with $R'^1$ and $R'^2$, which may be identical or different, representing a hydroxyl, $(C_1-C_4)$alkoxy or alkyl group, $R'^3$ representing a hydroxyl or $(C_1-C_4)$alkoxy group and $Z^1$ representing an oxygen or sulphur atom;

• a sterically hindered cyclic group, such as the adamantyl group; and
• optionally substituted alkoxy$(C_1-C_4)$alkyl.

**5.** Fluorescent dye according to any one of the preceding claims, in which Y represents an alkali metal or a protecting group chosen from:

➢ $(C_1-C_4)$alkylcarbonyl;
➢ arylcarbonyl;
➢ $(C_1-C_4)$alkoxycarbonyl;
➢ aryloxycarbonyl;
➢ aryl $(C_1-C_4)$alkoxycarbonyl;
➢ (di) $(C_1-C_4)$ (alkyl)aminocarbonyl;
➢ $(C_1-C_4)$ (alkyl) arylaminocarbonyl;
➢ optionally substituted aryl;
➢ 5- or 6-membered cationic monocyclic heteroaryl;
➢ 8- to 11-membered cationic bicyclic heteroaryl;
➢ cationic heterocycle of formula below:

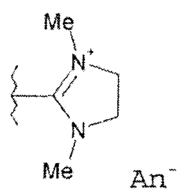

➢ isothiouronium $-C(NH_2)=N^+H_2$; $An^-$;
➢ isothiourea $-C(NH_2)=NH$; and
➢ $SO_3$ $M^+$ with $M^+$ representing an alkali metal or else M' of formula (II) and $M^+$ are absent.

**6.** Fluorescent dye according to any one of the preceding claims, belonging to formula (Ia) or (IIa) , which has the ethylene group linking the pyridinium part to the phenyl at the ortho- or para-position with respect to the pyridinium, i.e. at 2-4', 4-2', 4-4':

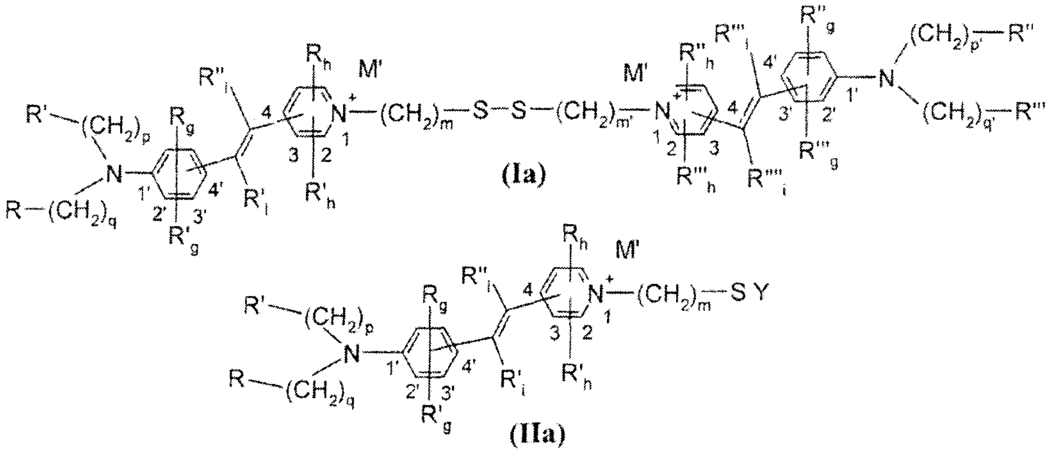

(Ia)

(IIa)

with R, R', R", R"', $R_g$, $R'_g$, $R''_g$, $R'''_g$, $R_h$, $R'_h$, $R''_h$, $R'''_h$, $R'_i$, $R''_i$, $R'''_i$, $R''''_i$, m, m', p, p', q, q', Y and M' being as defined in any one of the preceding claims.

**7.** Fluorescent dye according to any one of the preceding claims, chosen from the following dyes:

<table>
<tr><td colspan="2">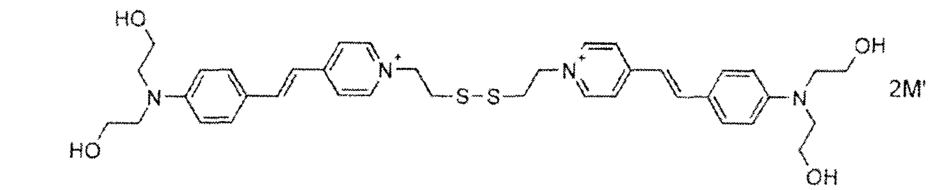</td></tr>
<tr><td colspan="2">1</td></tr>
<tr><td colspan="2">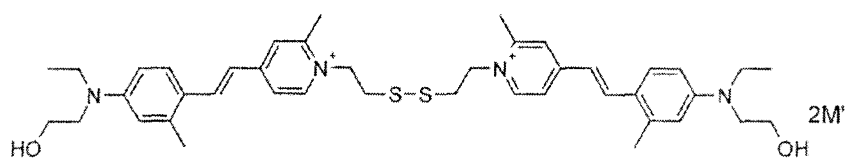</td></tr>
<tr><td colspan="2">2</td></tr>
<tr><td colspan="2">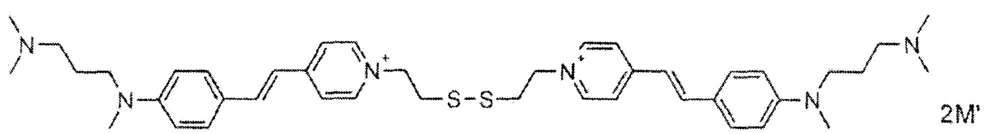</td></tr>
<tr><td colspan="2">3</td></tr>
<tr><td>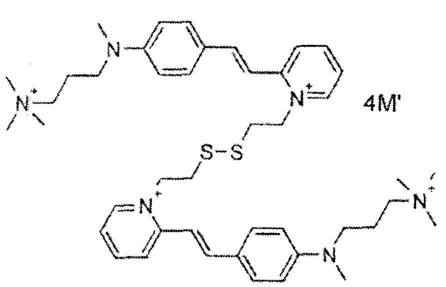</td><td>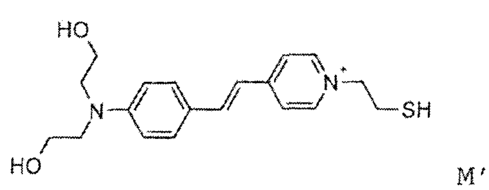</td></tr>
<tr><td>4</td><td>5</td></tr>
</table>

(continued)

| | |
|---|---|
| 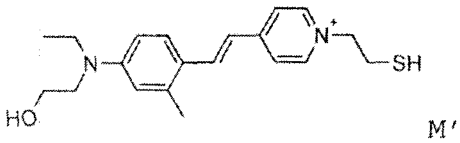 | 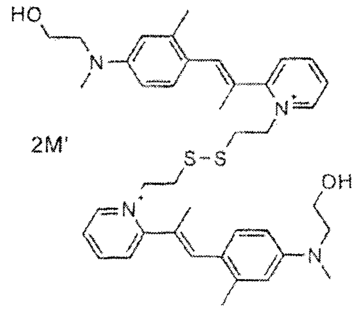 |
| **6** | **7** |
| 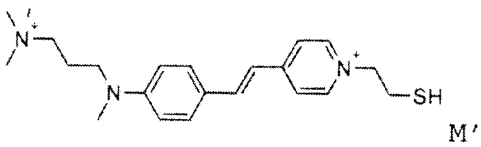 | 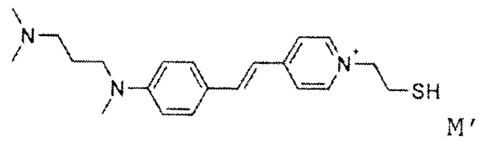 |
| **8** | **9** |
| 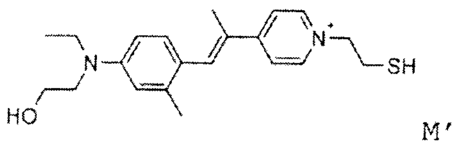 | 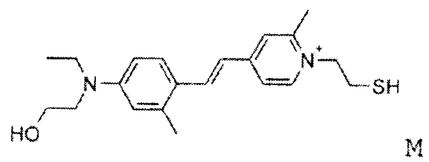 |
| **10** | **11** |
| 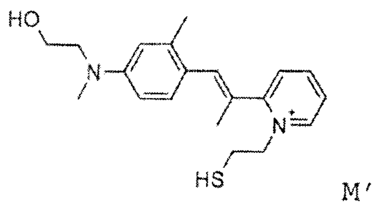 | 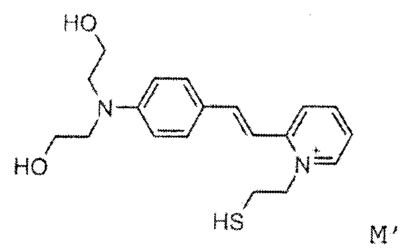 |
| **12** | **13** |
| 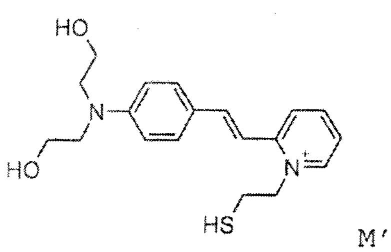 | 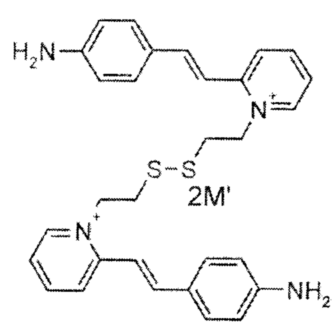 |
| **14** | **15** |

(continued)

| | |
|---|---|
| 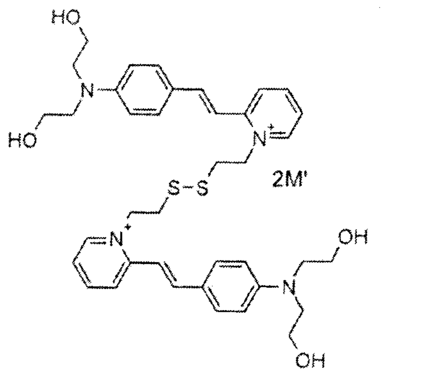 | 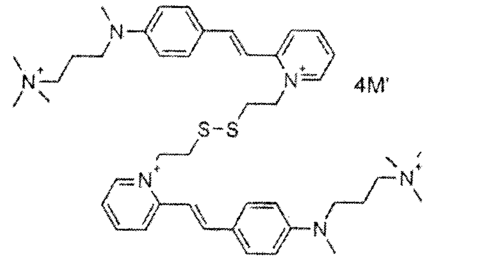 |
| **16** | **17** |
| 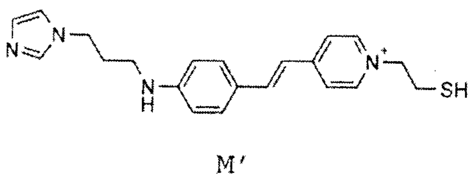 | 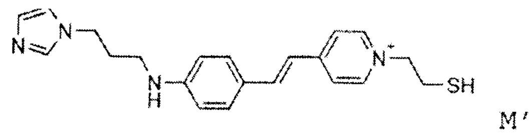 |
| **18** | **19** |
| 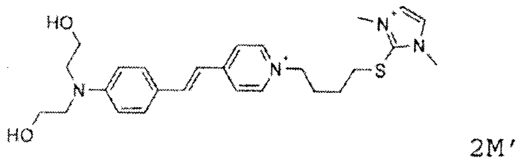 | 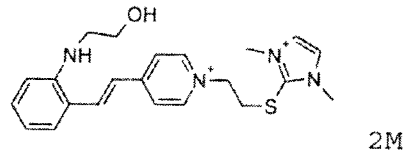 |
| **20** | **21** |
| 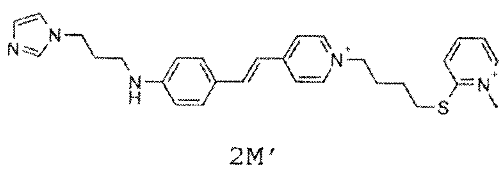 | 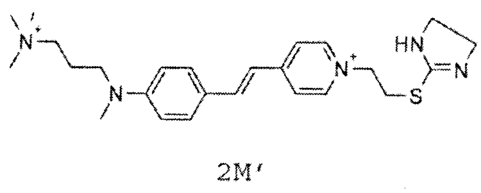 |
| **22** | **23** |
| 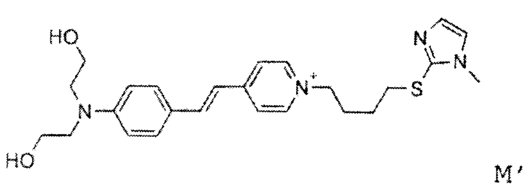 | 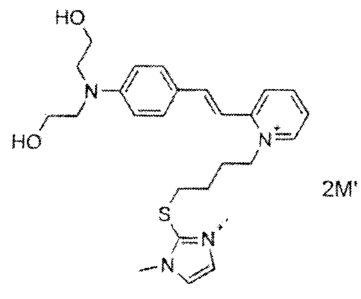 |
| **24** | **25** |

(continued)

| | |
|---|---|
| 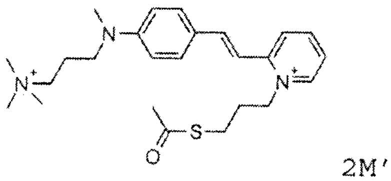 | 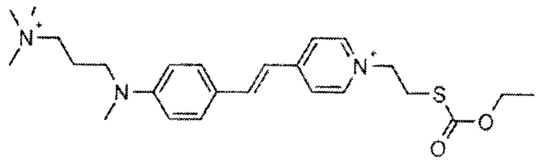 |
| **26** | **27** |
| 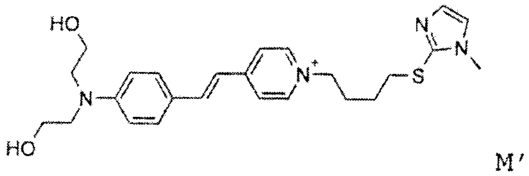 | 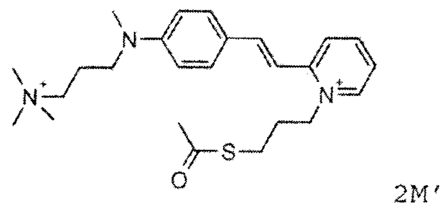 |
| **28** | **29** |
| 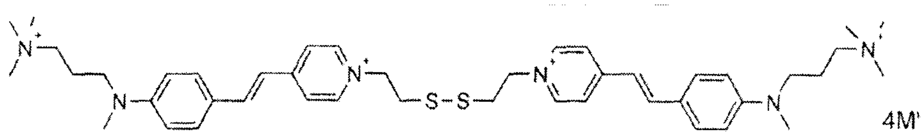 | |
| **30** | |
| 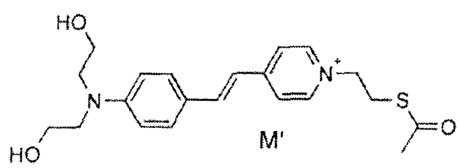 | |
| **31** | |
| 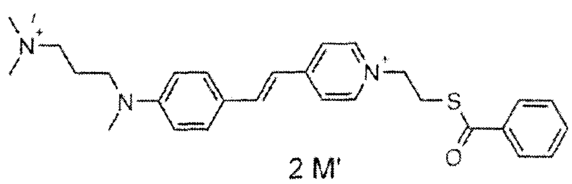 | |
| **32** | |

with M' representing an anionic counterion.

**8.** Dye composition comprising, in a suitable cosmetic medium, a fluorescent dye of formula (I) or (II) as defined in any one of Claims 1 to 7.

**9.** Dye composition comprising, in a suitable cosmetic medium, at least one fluorescent dye of formula (I) or (II) as defined in any one of Claims 1 to 7 and at least one reducing agent.

**10.** Dye composition according to the preceding claim, in which the reducing agent is chosen from cysteinee, homocysteinee, thiolactic acid, the salts of these thiols, phosphines, bisulphite, sulphites, thioglycolic acid, and also its esters, borohydrides and derivatives thereof, sodium salts, lithium, salts, potassium salts, calcium, salts, quaternary ammonium salts; and catechol borane.

**11.** Composition according to any one of Claims 8 to 10, in which the fluorescent dye of formula (I) or (II) is present in an amount of between 0.001% and 50% by weight, relative to the total weight of the composition.

**12.** Process for dyeing keratin materials, in which a dye composition as defined in any one of Claims 8 to 11, comprising, in a suitable cosmetic medium, at least one fluorescent dye of formula (I) or (II) as defined in any one of Claims 1 to 7, optionally in the presence of a reducing agent, is applied to the materials.

**13.** Dyeing process according to the preceding claim, in which, when the thiol, fluorescent dye of formula (II) comprises a protecting group Y, the application is preceded by a deprotection step.

**14.** Process for dyeing keratin materials according to either one of Claims 12 and 13, **characterized in that** the keratin materials are dark keratin fibres having a tone height of less than or equal to 6.

**15.** Process according to any one of Claims 12 to 14, in which the reducing agent is applied before or after the application of the fluorescent dye of formula (I) or (II) .

**16.** Process according to any one of Claims 12 to 15, in which the composition comprises an oxidizing agent.

**17.** Process according to any one of Claims 12 to 16, comprising an additional step consisting in applying an oxidizing agent to the keratin fibres.

**18.** Multicompartment device in which a first compartment contains a dye composition comprising a fluorescent dye of formula (I) or (II) as defined in Claims 1 to 7 and a second compartment contains a reducing agent .

**19.** Device according to the preceding claim, comprising a third compartment which contains an oxidizing agent.

**20.** Use of the fluorescent dyes of formula (I) or (II) as defined in Claims 1 to 7, for dyeing dark human keratin fibres.

**21.** Use according to the preceding claim, for lightening dark keratin fibres.

**22.** Use according to Claim 20 or 21, **characterised in that** the keratin fibres have a tone height of less than 6.

## Patentansprüche

**1.** Fluoreszierender Thiofarbstoff der Formel (I) oder (II):

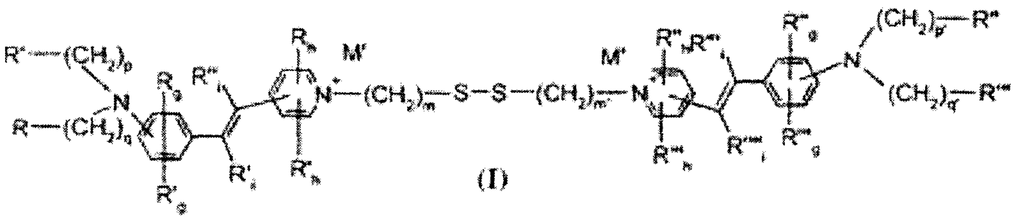

(I)

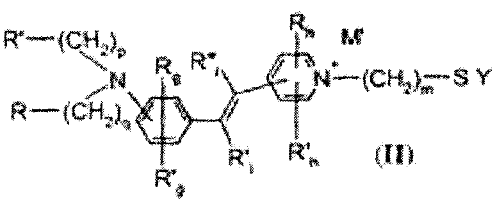

(II)

seine Salze mit einer organischen oder anorganischen Säure, optischen Isomere, geometrischen Isomere und Solvate wie Hydrate;

in den Formeln (I) und (II):

➢ die Gruppen R und R"', die gleich, oder verschieden sind, bedeuten eine Hydroxygruppe, eine Aminogruppe ($NR_aR_b$) oder eine Ammoniumgruppe ($N^+R_aR_bR_c$), $An^-$, wobei $R_a$, $R_b$ und $R_c$, die gleich oder verschieden sind, ein Wasserstoffatom oder eine ($C_1$-$_4$)Alkylgruppe bedeuten; oder zwei Alkylgruppen $R_a$ und $R_b$ der Aminogruppe oder Ammoniumgruppe bilden einen 5- bis 7-gliedrigen Heterocyclus, der gegebenenfalls ein weiteres Heteroatom enthält, das mit dem Stickstoffatom übereinstimmt oder davon verschieden ist; und An- bedeutet ein anionisches Gegenion;

➢ die Gruppen R' und R", die gleich oder verschieden sind, bedeuten ein Wasserstoffatom oder eine Gruppe, wie sie für R bzw. R'" definiert wurde;

➢ die Gruppen Rg, R'g, $R''_g$, $R'''_g$, $R_h$, $R'_h$, $R''_h$ und $R'''_h$, die gleich oder verschieden sind, bedeuten ein Wasserstoffatom, ein Halogenatom, eine Aminogruppe, eine (Di)($C_1$-$_4$)alkylaminogruppe, eine Cyanogruppe, eine Carboxygruppe, eine Hydroxygruppe, eine Trifluormethylgruppe, eine Acylaminogruppe, eine ($C_1$-$C_4$)Alkoxygruppe, eine ($C_{2-4}$) (Poly)hydroxyalkoxygruppe, eine ($C_1$-$C_4$)Alkylcarbonyloxygruppe, eine ($C_1$-$C_4$)Alkoxycarbonylgruppe, eine ($C_{1-4}$) Alkylcarbonylaminogruppe, eine Acylaminogruppe, eine Carbamoylgruppe, eine ($C_{1-}C_4$)Alkylsulfonylaminogruppe, eine Aminosulfonylgruppe, oder eine ($C_1$-$_{16}$)Alkylgruppe, die gegebenenfalls substituiert ist mit einer Gruppe, die ausgewählt ist unter ($C_{1-12}$)Alkoxy, Hydroxy, Cyano, Carboxy, Amino, (Di) ($C_{1-4}$)alkylamino, wobei die beiden Alkylgruppen, die von dem Stickstoffatom der Aminogruppe getragen werden, einen 5- bis 7-gliedrigen Heterocyclus bilden, der gegebenenfalls ein weiteres Heteroatom enthält, das mit dem Stickstoffatom übereinstimmt oder davon verschieden ist;

➢ die Gruppen $R'_i$, $R''_i$, $R'''_i$ und $R''''_i$, die gleich oder verschieden sind, bedeuten ein Wasserstoffatom oder eine ($C_{1-4}$)Alkylgruppe;

➢ m und m', die gleich oder verschieden sind, bedeuten eine ganze Zahl im Bereich von 1 bis 10 (Grenzen eingeschlossen);

➢ p, p', q und q', die gleich oder verschieden sind, bedeuten eine ganze Zahl im Bereich von 1 bis 6 (Grenzen eingeschlossen);

➢ M' ist ein anionisches Gegenion; und

➢ Y bedeutet: i) ein Wasserstoffatom; ii) ein Alkalimetall; iii) ein Erdalkalimetall; iv) eine Ammoniumgruppe: $N^+R^\alpha R^\beta R^\gamma R^\delta$ oder eine Phosphoniumgruppe: $P^+R^\alpha R^\beta R^\gamma R^\delta$, wobei $R^\alpha$, $R^\beta$, $R^\gamma$ und $R^\delta$, die gleich oder verschieden sind, ein Wasserstoffatom oder eine ($C_{1-4}$)Alkylgruppe bedeuten; oder v) eine Schutzgruppe für die Thiofunktion;

mit der Maßgabe, dass:

- wenn die Verbindung der Formel (I) oder (II) andere kationische Teile aufweist, sie mit einem oder mehreren anionischen Gegenionen kombiniert ist, mit denen die Elektroneutralität der Formel (I) oder (II) erreicht wird.

**2.** Fluoreszierender Farbstoff der Formel (II) nach dem vorhergehenden Anspruch, bei dem Y ein Wasserstoffatom oder ein Alkalimetall bedeutet.

**3.** Fluoreszierender Farbstoff der Formel (II) nach Anspruch 1, bei dem Y eine Schutzgruppe bedeutet.

**4.** Fluoreszierender Farbstoff der Formel (II) nach dem vorhergehenden Anspruch, bei dem Y eine Schutzgruppe bedeutet, die unter den folgenden Gruppen ausgewählt ist:

• ($C_{1-4}$)Alkylcarbonyl;
• ($C_{1-4}$)Alkylthiocarbonyl;
• ($C_{1-4}$)Alkoxycarbonyl;
• ($C_{1-4}$)Alkoxythiocarbonyl;
• ($C_{1-4}$)Alkylthio-thiocarbonyl;
• Di($C_{1-4}$)(alkyl)aminocarbonyl;
• Di(C 14)(alkyl)aminothiocarbonyl;
• Arylcarbonyl wie Phenylcarbonyl;
• Aryloxycarbonyl;
• Aryl($C_{1-4}$)alkoxycarbonyl;
• Di($C_{1-4}$)(alkyl)aminocarbonyl;
• ($C_{1-4}$)(Alkyl)arylaminocarbonyl;
• Carboxy;
• $SO_3^-$,$M^+$ mit $M^+$ bedeutet ein Alkalimetall oder M' der Formel (II) und $M^+$ fehlen;

• Aryl, gegebenenfalls substituiert;
• Heteroaryl, gegebenenfalls substituiert;
• Heterocycloalkyl, gegebenenfalls substituiert, gegebenenfalls kationisch;
• Isothiouronium $-C(NR'^{c}R'^{d})=N^{+}R'^{e}R'^{f}$, $An^{-}$, wobei $R'^{c}$, $R'^{d}$, $R'^{e}$ und $R'^{f}$, die gleich oder verschieden sind, ein Wasserstoffatom oder eine $(C_{1-4})$Alkylgruppe bedeuten; wobei $R'^{c}$ bis $R'^{f}$ vorzugsweise ein Wasserstoffatom bedeuten; und $An^{-}$ ein Gegenion ist;
• Isothioharnstoff $-C(NR'^{c}R'^{d})=NR'^{e}$, wobei $R'^{c}$, $R'^{d}$ und $R'^{e}$ die oben angegebenen Bedeutungen aufweisen;
• (Di)aryl$(C_{1-4})$alkyl, gegebenenfalls substituiert;
• (Di)heteroaryl$(C_{1-4})$alkyl, gegebenenfalls substituiert;
• $CR^{1}R^{2}R^{3}$, wobei $R^{1}$, $R^{2}$ und $R^{3}$, die gleich oder verschieden sind, ein Halogenatom oder eine Gruppe bedeuten, die ausgewählt ist unter:

- $(C_{1-4})$Alkyl;
- $(C_{1-4})$Alkoxy;
- Aryl, gegebenenfalls substituiert;
- Heteroaryl, gegebenenfalls substituiert;
- $P(Z^{1})R'^{1}R'^{2}R'^{3}$, wobei $R'^{1}$ und $R'^{2}$, die gleich oder verschieden sind, Hydroxy, $(C_{1-4})$Alkoxy oder Alkyl bedeuten, $R'^{3}$ Hydroxy oder $(C_{1-4})$Alkoxy ist und $Z^{1}$ ein Sauerstoffatom oder ein Schwefelatom bedeutet;

• cyclisch sterisch gehindert; wie die Adamantylgruppe; und
• Alkoxy$(C_{1-4})$alkyl, gegebenenfalls substituiert.

**5.** Fluoreszierender Farbstoff nach einem der vorhergehenden Ansprüche, bei dem Y ein Alkalimetall oder eine Schutzgruppe bedeutet, die ausgewählt ist unter:

➢ $(C_{1-4})$Alkylcarbonyl;
➢ Arylcarbonyl;
➢ $(C_{1-4})$Alkoxycarbonyl,
➢ Aryloxycarbonyl;
➢ Aryl$(C_{1-4})$alkoxycarbonyl;
➢ Di$(C_{1-4})$(alkyl)aminocarbonyl;
➢ $(C_{1-4})$(Alkyl)arylaminocarbonyl;
➢ Aryl, gegebenenfalls substituiert;
➢ 5-, 6-gliedriges kationisches monocyclisches Heteroaryl;
➢ 8- bis 11 -gliedriges kationisches bicyclisches Heteroaryl;
➢ dem kationischen Heterocyclus der folgenden Formel:

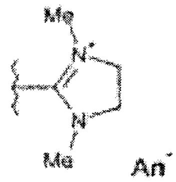

➢ Isothiouronium $-C(NH_{2})=N^{+}H_{2}$, $An^{-}$;
➢ Isothioharnstoff $-C(NH_{2})=NH$; und
➢ $SO_{3}^{-}$,$M^{+}$ mit $M^{+}$ bedeutet ein Alkalimetall oder M' der Formel (II) und $M^{+}$ fehlen.

**6.** Fluoreszierender Farbstoff nach einem der vorhergehenden Ansprüche, der zur Formel (Ia) oder (IIa) gehört und eine Ethylengruppe aufweist, die den Pyridiniumteil mit dem Phenylteil in ortho- oder para-Stellung des Pyridinium verbindet, d.h. 2-4', 4-2', 4-4':

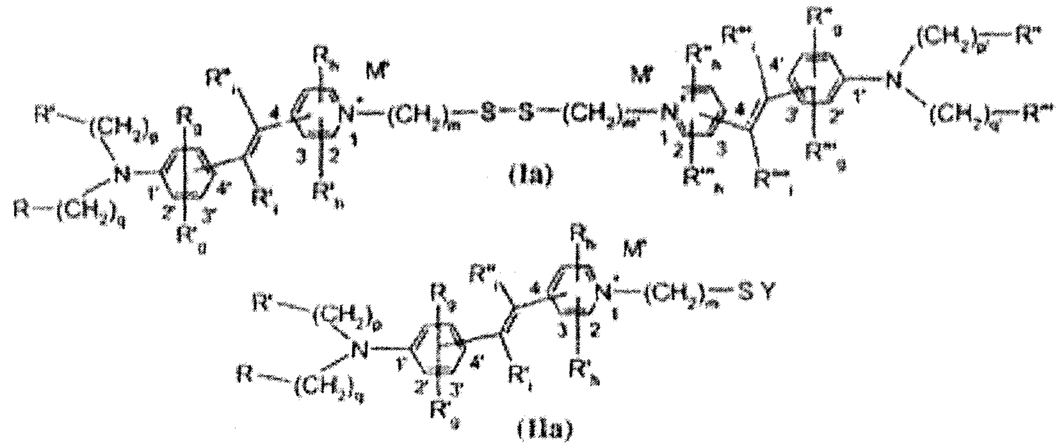

(Ia)

(IIa)

wobei R, R’, R", R"’, $R_g$, $R'_g$, R"g, $R'''_g$, $R_h$, $R'_h$, $R''_h$, $R'''_h$, $R''_i$, $R'''_i$ und $R''''_i$, m, m’, p, p’, q, q’, Y und M’ die in einem der vorhergehenden Ansprüchen definieren Bedeutungen aufweisen.

7. Fluoreszierender Farbstoff nach einem der vorhergehenden Ansprüche, der unter den folgenden Farbstoffen ausgewählt ist:

<table>
<tr><td colspan="2">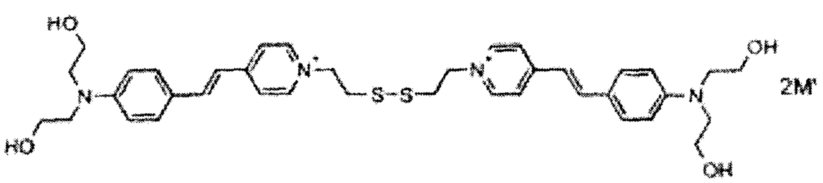</td></tr>
<tr><td colspan="2">1</td></tr>
<tr><td colspan="2">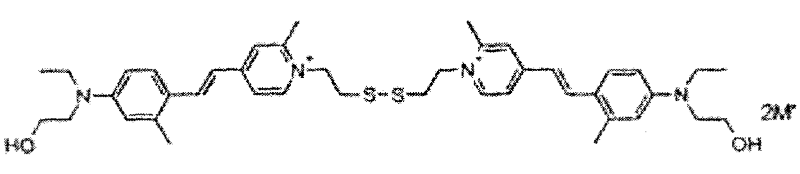</td></tr>
<tr><td colspan="2">2</td></tr>
<tr><td colspan="2">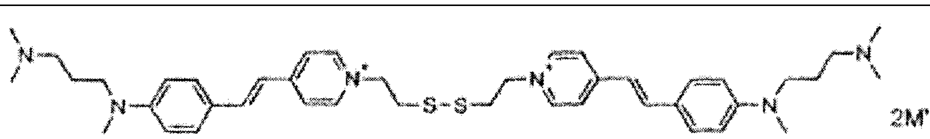</td></tr>
<tr><td colspan="2">3</td></tr>
<tr><td>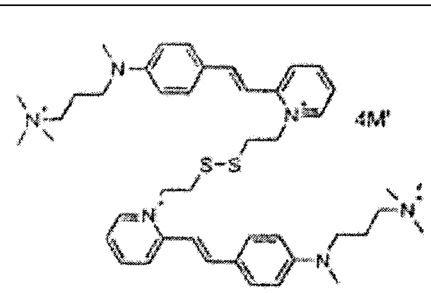</td><td>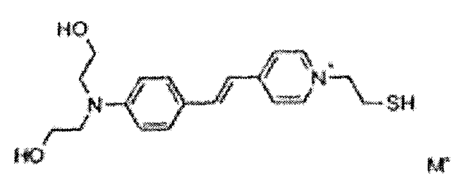</td></tr>
<tr><td>4</td><td>5</td></tr>
</table>

(fortgesetzt)

| | |
|---|---|
| 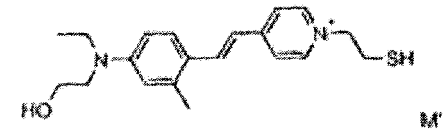 | 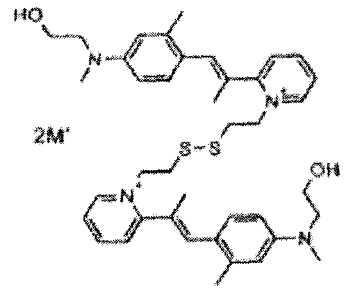 |
| **6** | **7** |
| 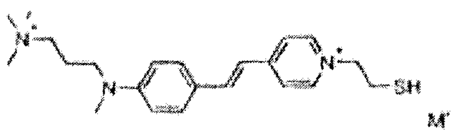 | 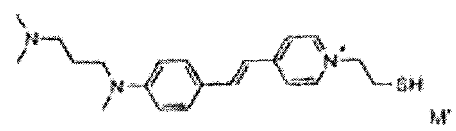 |
| **8** | **9** |
| 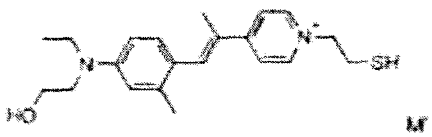 | 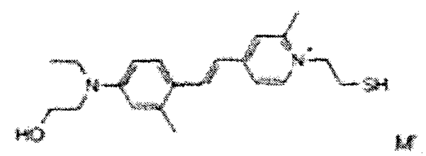 |
| **10** | **11** |
| 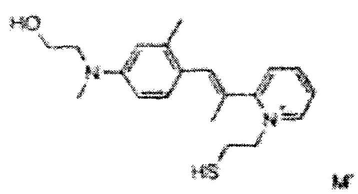 | 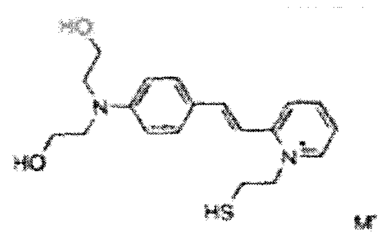 |
| **12** | **13** |
| 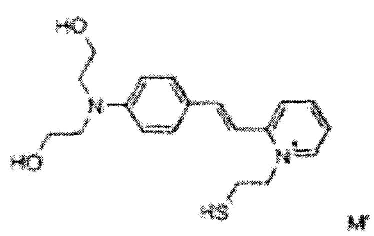 | |
| **14** | |

(fortgesetzt)

| | |
|---|---|
| 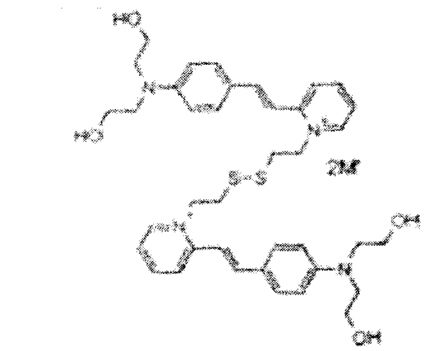 | 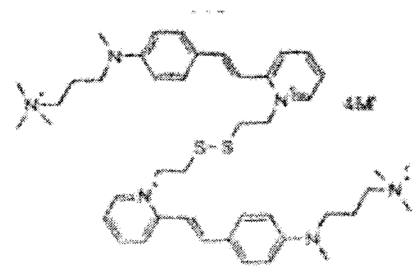 |
| **16** | **17** |
| 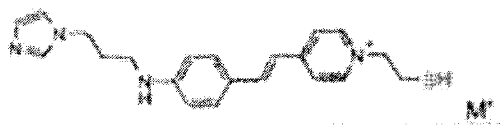 | 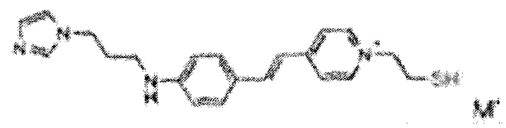 |
| **18** | **19** |
| 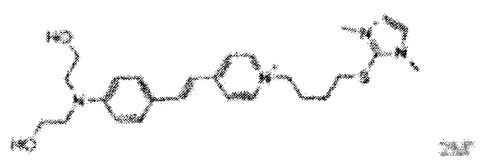 | 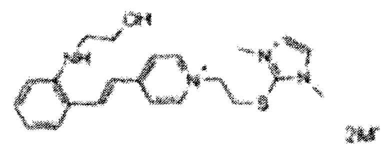 |
| **20** | **21** |
| 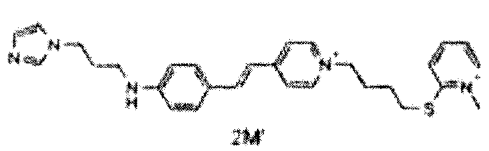 | 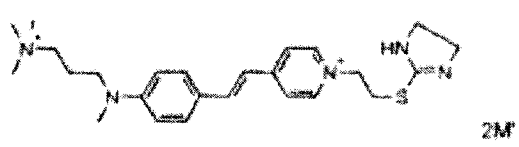 |
| **22** | **23** |
| 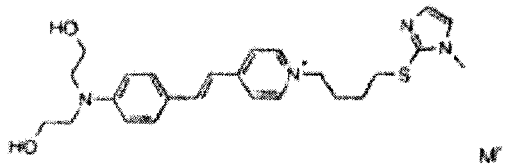 | 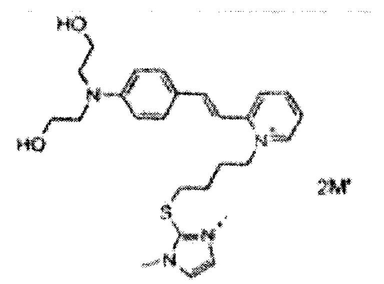 |
| **24** | **25** |
| 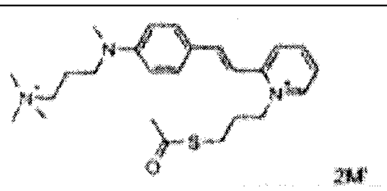 | 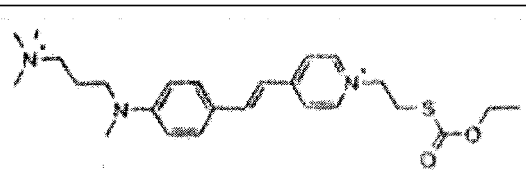 |
| **26** | **27** |

(fortgesetzt)

| | |
|---|---|
| 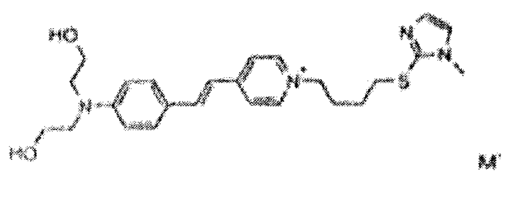 | 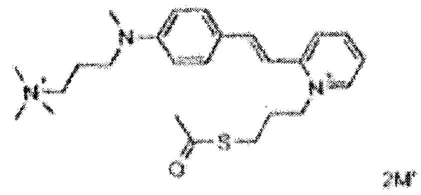 |
| **28** | **29** |
| 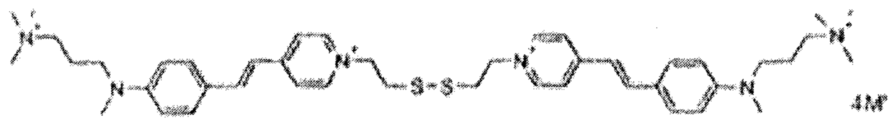 | |
| **30** | |
| 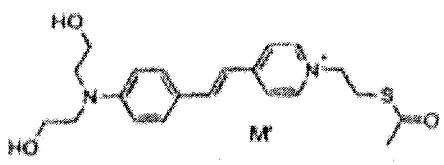 | |
| **31** | |
| 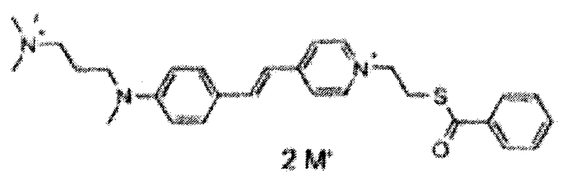 | |
| **32** | |

wobei M' ein anionisches Gegenion bedeutet.

**8.** Farbmittelzusammensetzung, die in einem kosmetisch geeigneten Medium einen fluoreszierenden Farbstoff der Formel (I) oder (II) enthält, wie er in einem der Ansprüche 1 bis 7 definiert ist.

**9.** Farbmittelzusammensetzung, die in einem kosmetisch geeigneten Medium mindestens einen fluoreszierenden Farbstoff der Formel (I) oder (II), wie er in einem der Ansprüche 1 bis 7 definiert ist, und mindestens ein Reduktionsmittel enthält.

**10.** Farbmittelzusammensetzung nach dem vorhergehenden Anspruch, wobei das Reduktionsmittel unter Cystein, Homocystein, Thiomilchsäure, den Salzen dieser Thiole, Phosphinen, Bisulfit, Sulfiten, Thioglycolsäure, sowie deren Estern, Borhydriden und ihren Derivaten, Natriumsalze, Lithiumsalzen, Kaliumsalzen, Calciumsalzen, quartären Ammoniumverbindungen; Catecholboran ausgewählt ist.

**11.** Farbmittelzusammensetzung nach einem der Ansprüche 8 bis 10, wobei der fluoreszierenden Farbstoff der Formel (I) oder (II) in einem Mengenanteil von 0,001 bis 50 Gew.-%, bezogen auf das Gesamtgewicht der Zusammensetzung, enthalten ist.

**12.** Verfahren zum Färben von Keratinsubstanzen, wobei auf die Keratinsubstanzen eine Farbmittelzusammensetzung aufgetragen wird, wie sie in einem der Ansprüche 8 bis 10 definiert ist und die in einem kosmetisch geeigneten Medium mindestens einen fluoreszierenden Farbstoff der Formel (I) oder (II), wie er in einem der Ansprüche 1 bis 7 definiert ist, gegebenenfalls in Gegenwart eines Reduktionsmittels enthält.

**13.** Verfahren zum Färben nach dem vorhergehenden Anspruch, bei dem, wenn der fluoreszierenden Thiofarbstoff' der Formel (II) eine Schutzgruppe Y aufweist, die Schutzgruppe vor dem Auftragen entfernt wird.

14. Verfahren zum Färben von Keratinsubstanzen nach einem der Anspruche in oder 13, **dadurch gekennzeichnet, dass** die Keratinsubstanzen dunkle Keratinfasern sind, die einen Farbton von 6 oder darunter aufweisen.

15. Verfahren nach einem der Ansprüche 12 bis 14, bei dem das Reduktionsmittel vor oder nach dem Auftragen des fluoreszierenden Farbstoffes der Formel (I) oder (II) aufgebracht wird.

16. Verfahren nach einem der Ansprüche 12 bis 15, bei dem die Zusammensetzung ein Oxidationsmittel enthält.

17. Verfahren nach einem der Ansprüche 12 bis 16, das einen weiteren Schritt umfasst, der darin besteht, ein Oxidationsmittel auf die Keratinfasern aufzubringen.

18. Vorrichtung mit mehreren Abteilungen, wobei eine Abteilung eine Farbmittelzusammensetzung enthält, die einen fluoreszierenden Farbstoff der Formel (I) oder (II), wie er in einem der Ansprüche 1 bis 7 definiert ist, aufweist, und eine zweite Abteilung ein Reduktionsmittel enthält.

19. Vorrichtung nach dem vorhergehenden Anspruch, die eine dritte Abteilung aufweist, welche ein Oxidationsmittel enthält.

20. Verwendung von fluoreszierenden Farbstoffen der Formel (I) oder (II), wie sie in einem der Ansprüche 1 bis 7 definiert sind, zum Färben von dunklen menschlichen Keratinfasern.

21. Verwendung nach dem vorhergehenden Anspruch zum Aufhellen von dunklen Keratinfasern.

22. Verwendung nach Anspruch 20 oder 21, **dadurch gekennzeichnet, dass** die Keratinfasern einen Farbton unter 6 aufweisen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2830189
- WO 2004091473 A
- CA 2024509
- WO 9951194 A
- FR 1156407
- WO 2005097051 A
- FR 2586913


### Littérature non-brevet citée dans la description


- **Guise ; Stapleton.** *Journal of the Society of Dyers and Colourists,* 1975, vol. 91, 259-264
- *Journal of Cosmetic Chemistry,* 1991, vol. 42, 1-17
- **Charles ZVIAK.** Science des traitement capillaires. Ed. Masson, 1988, 215, 278
- **T. W. Greene.** Protective Groups in Organic Synthesis. John Willey & Sons, 1981, 193-217
- **P. Kocienski.** Protecting Groups. Thieme, 2005
- « Thiols and organic Sulfides », « Thiocyanates and Isothiocyanates, organic ». Ullmann's Encyclopedia. Wiley-VCH, 2005
- Reactions, Mechanisms and Structures. **J. March.** Advanced Organic Chemistry. John Willey & Sons, 1992
- **T. W. Greene.** *Protective Groups in Organic Synthesis*
- **T. W. Greene.** Protective Groups in Organic Synthesis. John Willey & Sons, 1981